# EUROPEAN PATENT APPLICATION

(11) **EP 1 890 146 A1**
(43) Date of publication of application: **20.02.2008**
(21) Application number: 06756902.0
(22) Date of filing: 01.06.2006
(51) Int. Cl.: G01N 33/53, C12N 15/09, C12Q 1/68

(54) **METHOD FOR DETECTING OF NUCLEIC ACID**

(30) Priority: 01.06.2005 JP 2005161560; 15.06.2005 JP 2005175208
(71) Applicant: Olympus Corporation, Tokyo 151-0072 (JP)
(72) Inventor: KONDO, Seiji, c/o Olympus Intellectual Property Services Co.,Ltd, Hachioji-shi, Tokyo 192-8512 (JP); HASHIDO, Kento, c/o Olympus Intellectual Property Services Co.,Ltd, Hachioji-shi, Tokyo 192-8512 (JP)
(74) Representative: Wibbelmann, Jobst
(86) International application number: PCT/JP2006/311025
(87) International publication number: WO 2006/129770

(57) **Abstract**

The present invention provides a method for detecting a nucleic acid by specific binding between ligand and receptor, in particular, a method of detecting a SNP by the specific binding between a ligand and receptor. The present invention also provides a method for detecting a nucleic acid that is simpler, requiring only a single measurement operation, and shorter in measurement period. The present invention also provides a highly sensitive and highly accurate method for detecting a nucleic acid by using a specific binding reaction between a ligand and receptor in a reaction at the interface of a liquid and solid. The present invention also detects a nucleic acid by using a coagulation reaction of dispersible particles.

SEQUENCE LISTING

<110>  Olympus Corporation

<120>  A method for detecting nucleic acids

<130>  06S0589P

<150>  JP 2005-161560
<151>  2005-06-01

<150>  JP 2005-175208
<151>  2005-06-15

<160>  25

<170>  PatentIn version 3.2

<210>  1
<211>  23
<212>  PRT
<213>  Human

<400>  1

Ser Thr Asp Ala Thr Ser Ile Leu Gly Ile Gly Thr Val Leu Asp Gln
1               5                   10                  15

Ala Glu Thr Ala Gly Ala Arg

            20

<210> 2
<211> 25
<212> PRT
<213> Human

<400> 2

Pro Pro Gly Ser Val Thr Val Pro His Pro Asn Ile Glu Glu Val Ala
1               5                   10                  15

Leu Ser Thr Thr Gly Glu Ile Pro Phe
            20                  25

<210> 3
<211> 16
<212> PRT
<213> Human

<400> 3

Tyr Tyr Arg Gly Leu Asp Val Ser Val Ile Pro Thr Ser Gly Asp Val
1               5                   10                  15

<210> 4

<211>  18

<212>  DNA

<213>  Human

<400>  4

ctgtctgacg agatagca                                                    18

<210>  5

<211>  18

<212>  DNA

<213>  Human

<400>  5

ctgtctgacg agatagcg                                                    18

<210>  6

<211>  38

<212>  DNA

<213>  Human

<400>  6

acactagtaa atcacagcac tcttacaggt catgagaa                              38

<210>  7

<211>  298

<212>  DNA

<213>  Human

<400> 7

caaatcttcc agcactgact ggtttcaccg aactgcataa cttctacctg cttacacata 60

acacaagctt ctttgaccat tttttggaac tcacaattta kactagacct catggttgcc 120

ctgtccttgg atacctcctg actgggaagc tgtctaggcc tttatcttac tgcagctgtt 180

aggcagtcat agaggttgac aggcaggcca cctgtctgac gagatagcrc aggtcaggtg 240

ggctcactct aagcccctga ttctcatgac ctgtaagagt gctgtgattt actagtgt 298

<210> 8

<211> 18

<212> DNA

<213> Human

<400> 8

ctgtctgacg agatagca 18

<210> 9

<211> 18

<212> DNA

<213> Human

<400> 9

ctgtctgacg agatagcg 18

<210> 10

<211> 18

<212> DNA

<213> Human

<400> 10

caggtcaggt gggctcac 18

<210> 11

<211> 45

<212> DNA

<213> Human

<400> 11

acctgcttac acataacaca agcttctttg accatttttt ggaac 45

<210> 12

<211> 38

<212> DNA

<213> Human

<400> 12

acactagtaa atcacagcac tcttacaggt catgagaa 38

<210> 13

<211> 18

<212>  DNA

<213>  Human

<400>  13

ctgtctgacg agatagca                                                    18

<210>  14

<211>  18

<212>  DNA

<213>  Human

<400>  14

ctgtctgacg agatagcg                                                    18

<210>  15

<211>  18

<212>  DNA

<213>  Human

<400>  15

caggtcaggt gggctcac                                                    18

<210>  16

<211>  18

<212>  DNA

<213>  Human

<400> 16

ctgtctgacg agatagca 18

<210> 17

<211> 18

<212> DNA

<213> Human

<400> 17

ctgtctgacg agatagcg 18

<210> 18

<211> 18

<212> DNA

<213> Human

<400> 18

caggtcaggt gggctcac 18

<210> 19

<211> 51

<212> DNA

<213> Human

<400> 19

ctcccctcag aacataacat agtaatgcgg taagtcgcat aaaaaccatt c 51

<210> 20

<211> 51

<212> DNA

<213> Human

<400> 20

ttcccctcag aacataacat agtaatgcgg taagtcgcat aaaaaccatt c 51

<210> 21

<211> 48

<212> DNA

<213> Human

<400> 21

gtgaaaattc ccctaattcg atgaggtcgg cgcatagctg ataacaat 48

<210> 22

<211> 48

<212> DNA

<213> Human

<400> 22

ttgaaaattc ccctaattcg atgaggtcgg cgcatagctg ataacaat 48

<210> 23

<211> 24

<212> DNA

<213> Human

<400> 23

gcttatcttt ccctttattt ttgc 24

<210> 24

<211> 20

<212> DNA

<213> Human

<400> 24

gctgatcggc aaggtgttct 20

<210> 25

<211> 171

<212> DNA

<213> Human

<400> 25

gcttatcttt ccctttattt ttgctgcggt aagtcgcata aaaaccattc ttcataattc 60

aatccattta ctatgttatg ttctgagggg atgaaaattc ccctaattcg atgaagattc 120

ttgctcaatt gttatcagct atgcgccgac cagaacacct tgccgatcag c 171

## Description

### Technical Field

The present invention relates to a method for detecting a nucleic acid. Specifically, it relates to a method for detecting a nucleic acid by specific binding between a ligand and receptor. More specifically, it relates to a method for detecting a nucleic acid by using a coagulation reaction of dispersible particles.

### Background Art

Recently, there is an increasing need for analysis of single-nucleotide polymorphisms (SNPs) for realization of personalized medicine. Generally, SNP analysis is performed by microarray technology. For example, AmpliChip P450, available from Roche Diagnostic, is a typical SNP analyzer that is approved by the FDA as an SNP diagnostic kit.

However, nucleic acid detection on microarray is performed based on hybridization between nucleic acids. Hybridization between nucleic acids generally demands a high-temperature reaction condition of 45°C or higher, and the reaction condition varies significantly according to the GC content in the nucleotide sequence of the nucleic acid in reaction and the length of the nucleic acid, and thus it is difficult to consistently perform hybridization under an optimized reaction condition. Alternatively, detection of a nucleic acid on microarray is performed by hybridization of a sample nucleic acid in liquid solution with a labeled nucleic acid on a solid base plate. It is difficult to adjust the condition for a reaction proceeding at the interface of a liquid and solid because the chemical states are significantly different from each other. As a result, such a reaction often causes problems, such as of insufficient reaction and false reaction.

In contrast, specific reactions between ligands and receptors, including antigen-antibody reactions, are advantageously easier in adjusting the reaction conditions, and the sensitivity and the reproducibility of the reactions are higher, compared to the hybridization reaction. An array which makes use of such a specific reaction between a ligand and receptor is disclosed, for example, in PCT National Publication No. 2003-535569. However, the method demands two measurement operations; solution hybridization and endonuclease reaction, and is thus complicated in operation and demands an extended period of time for measurement. In addition, it is also needed to perform an enzyme reaction. The enzyme reaction, which is carried out with a nucleic acid on a solid base plate, demands a reaction at the interface of a liquid and solid, as described above, causing the problem of insufficient reaction and false reaction.

### Disclosure of Invention

An object of the present invention is to provide a method for detecting a nucleic acid by specific binding between a ligand and receptor; in particular, a method for detecting a SNP by specific binding between a ligand and receptor. Another object of the present invention is to provide a method for detecting a nucleic acid that is easier, requiring only one measurement operation, and shorter in the measuring period. A further object of the present invention is to provide a highly sensitive and highly accurate method for detecting a nucleic acid by using a specific binding reaction between a ligand and receptor occurring at the interface of a liquid and solid. A further object of the present invention is to provide a method for detecting a nucleic acid by using a coagulation reaction of dispersible particles.

A method for detecting a nucleic acid according to one aspect of the present invention comprises: (1) binding first and second ligands to a nucleic acid to be detected; (2) in the nucleic acid to which the ligands have been bound, binding the first ligand to a solid phase carrier supporting receptors specifically binding to the first ligand, and obtaining a complex of the nucleic acid - solid phase carrier; (3) in the complex, binding the second ligand to a receptor modified with a labeling substance specifically binding to the second ligand; and (4) detecting the nucleic acid by detecting the labeling substance.

A method for detecting a nucleic acid according to another aspect of the present invention comprises: (1) reacting the target nucleic acid to which different kinds of first and second ligands have been attached, with first particles to which two or more receptors specifically binding to the first ligand have been attached and second particles to which two or more receptors specifically binding to the second ligand have been attached; and (2) obtaining an aggregate in such a manner that the first and second particles are mutually linked when the target nucleic acid is bound to the first and second particles, and a large number of the particles are mutually linked when two or more target nucleic acids are bound to one of the particles, and simultaneously bound to the other particles respectively, and measuring the aggregate by spectrophotometry.

The method for detecting a nucleic acid according to the present invention allows highly sensitive and highly accurate detection of a nucleic acid, consequently assuring high reproducibility of the measurement results. In addition, use of the specific binding between ligand and receptor, instead of hybridization reaction, enables reduction in temperature of the reaction condition to a low temperature of around 37°C. The method, which requires only a single measurement operation, is simpler in operation and allows measurement in a shorter period of time, consequently leading to reduction in cost.

Further, detection of a nucleic acid by using the coagulation reaction of a dispersion polymer allows simpler and highly accurate detection of nucleic acids and consequently assures higher reproducibility of the measurement results.

### Brief Description of Drawings

FIG. 1 is a schematic view showing the step of binding a ligand to a nucleic acid.
FIG. 2 is a schematic view showing the step of binding a ligand to a nucleic acid under a competitive reaction condition.
FIG. 3 is a schematic view showing the step of binding different ligands to multiple kinds of nucleic acids.
FIG. 4 is a schematic view showing a method for detecting a ligand-modified nucleic acid.
FIG. 5 is a schematic view showing a step for detecting a ligand-modified nucleic acid.
FIG. 6 shows the amino acid sequence of a ligand according to the present invention.
FIG. 7 shows the nucleotide sequence of a primer according to the present invention.
FIG. 8 shows the nucleotide sequence of a target nucleic acid.
FIG. 9 shows the nucleotide sequence of a nucleic acid probe according to the present invention.
FIG. 10 shows the nucleotide sequence of a primer in an Example.
FIG. 11 shows the nucleotide sequence of a nucleic acid probe in an Example.
FIG. 12 is a bar chart showing the results of Examples.
FIG. 13 is a bar chart showing the results of Modifications.
FIG. 14 is a schematic view of a ligand-bound target nucleic acid.
FIG. 15 is a schematic view illustrating receptor-bound dispersive fine particles.
FIG. 16 is a schematic view illustrating aggregation of fine particles.
FIG. 17 is a schematic view illustrating fine particle aggregates during analysis by a spectrophotometric method.
FIG. 18 is a schematic view showing the principle of the method of detecting nucleic acid mutation by a PCR-SSP method.
FIG. 19 is a schematic view showing the principle of the method of detecting nucleic acid mutation by ligation.

### Best Mode for Carrying Out the Invention

Hereinafter, the present invention will be described in detail. In the first half of the description, described is a method of detecting a nucleic acid by specific binding between a ligand and receptor according to the present invention. In the latter half of the description, a method of detecting a nucleic acid by specific binding between a ligand and receptor according to the present invention, in particular a method of detecting a nucleic acid by using coagulation reaction of dispersion particles, will be described.

### I. The method of detecting a nucleic acid with specific binding between ligand and receptor according to the present invention

Hereinafter, steps of the present invention, specifically steps of modifying the target nucleic acid with a ligand substance and of detecting a ligand-modified nucleic acid, will be described in detail.

### 1. Step of modifying a target nucleic acid with a ligand substance

### (1) Detection of a single target nucleic acid

The target nucleic acid means a particular nucleic acid molecule present in a sample. The nucleic acids to be detected include all kinds of DNA and RNA such as cDNA, genome DNA, synthetic DNA, mRNA, total RNA, hnRNA, synthetic RNA. In particular, in the present invention, a particular nucleic acid molecule present in a biological sample is detected.

When the target nucleic acid means a single nucleotide, the first ligand means a substance specifically binding to a receptor supported on a solid-phase carrier. Similarly, the second ligand means a substance specifically binding to a receptor modified with a labeling substance.

The ligand substances include all substances usable in the present invention, such as hapten, biotin, dioxygen, fluorescein, Alexa, carbohydrates, peptides, proteins, polyhistidine, antigen/antibody substances, HA, GST, Tap, and Flag. Biotin, dioxygen, fluorescein, Alexa and others are commercially available and relatively inexpensive. Carbohydrates and peptides are higher in the degree of freedom in designing the chemical structure, and various kinds of haptens are easily prepared and can be used after being properly selected as needed. The hapten is preferably a hydrophilic organic compound; the carbohydrate is preferably an oligosaccharide having two or more monosaccharides; and the peptide is preferably an oligopeptide having six or more amino acid residues. Antigen and antibody substances may be used for detection of a nucleic acid by immune reaction. It is possible to detect specific binding between a ligand and receptor by such an antigen-antibody reaction.

The labeling substances described above include substances known by those skilled in the art. In particular, optical markers that generate an optical signal such as fluorescence or luminescence (chemoluminescence or bioluminescence) are preferable.

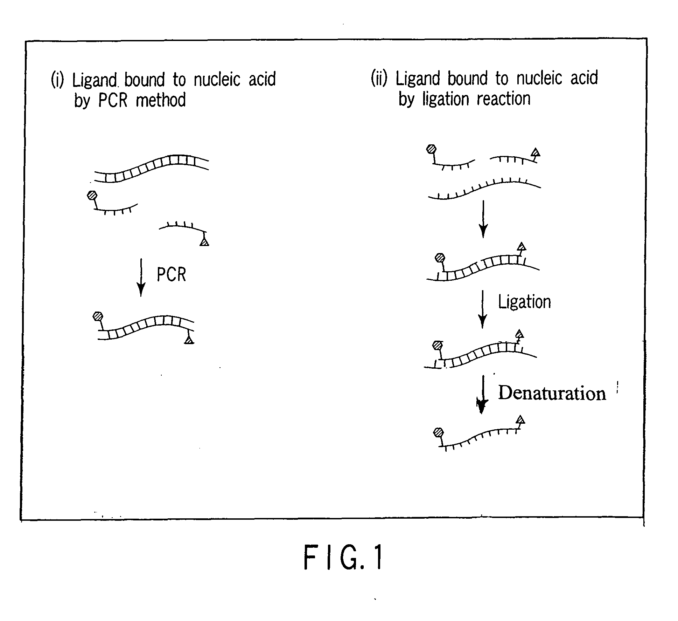

The methods of binding first and second ligands to the target nucleic acid include a method of performing a polymerase chain reaction (PCR) by using a ligand-modified primer (FIG. 1(i)), a method of performing a ligation reaction on the target nucleic acid by using a ligand-modified nucleic acid probe (FIG. 1(ii)), and the like. Use of the PCR method enables modification of the target nucleic acid with a ligand and amplification of the resulting nucleic acid (FIG. 1(i)). In performing the ligation reaction, it is preferable to amplify the nucleic acid region for the ligation reaction previously by the PCR method. It is thus possible to prevent binding of the nucleic acid probe to similar sequences present in the other region of the nucleic acid and to perform the ligation reaction with high accuracy. Ligation reaction in the nucleotide region amplified and subsequent denaturation give a ligand-modified nucleic acid (FIG. 1(ii)).

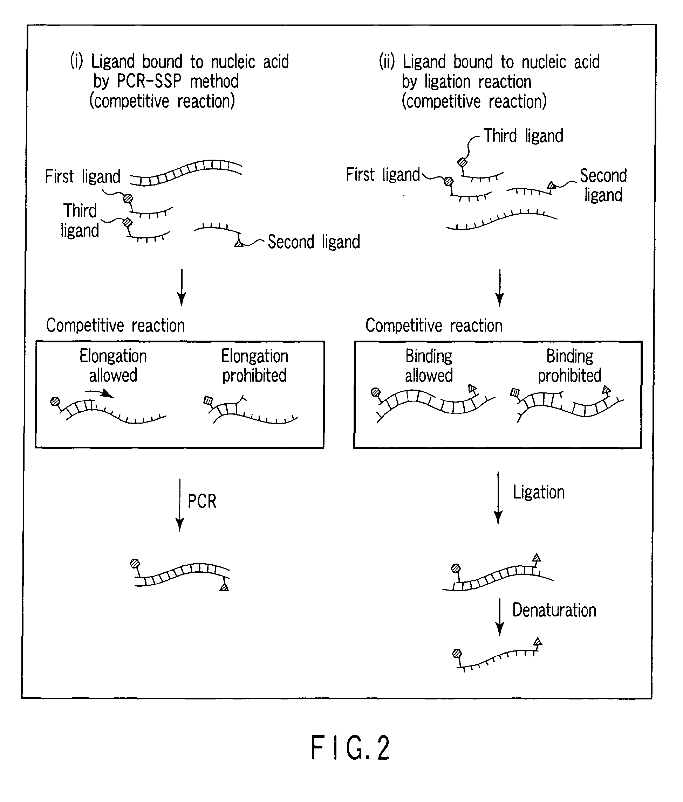

As shown in FIG. 2(i), it is also possible to bind a ligand to a nucleic acid by a PCR-SSP method by using a competitive reaction. Specifically, a primer similar to the first ligand-modified primer is prepared in advance. The primer can bind to the target nucleic acid at the same site as that of the first ligand-modified primer, but the polymerase elongation reaction does not proceed because the elongation-sided terminal is mismatched. It is possible to modify the target nucleic acid with a ligand accurately by the PCR method with a primer having such a pseudo-sequence. FIG. 2(ii) shows a method of binding a ligand to a nucleic acid by a ligation reaction, similarly by using a competitive reaction. A nucleic acid probe similar to the first ligand-modified nucleic acid probe is prepared in advance. The nucleic acid probe can bind to the target nucleic acid at the same site as that of the first ligand-modified primer, but the polymerase ligation reaction does not proceed because the binding-sided terminal is mismatched. It is possible to improve the accuracy of the ligation reaction by performing the ligation reaction with a nucleic acid probe having such a pseudo-sequence. Denaturation after completion of ligation gives a nucleic acid modified with first and second ligands. It is then possible to obtain a nucleic acid modified with the first and second ligands and a nucleic acid modified with the second and third ligands simultaneously in the same solution, by modifying the primer or probe modified with a first ligand and the primer or probe having a pseudo-sequence with a third ligand. In such a case, it is possible to perform measurement at higher accuracy without being influenced by the concentration of the target nucleic acid and the nonspecific reaction of the probe, by detecting the first ligand-modified nucleic acid and the third ligand-modified nucleic acid separately and determining the ratio in amounts of the first ligand and the third ligand.

The first and second ligands may be the same as or different from each other. In addition, the first or second ligand need not be a single molecule and may contain two or more molecules.

### (2) Detection of multiple target nucleic acids

Detection of multiple kinds of nucleic acid by reaction between ligand and receptor requires modification of the multiple kinds of nucleic acids respectively with different combinations of ligands. Although there are various methods available for determining the relationship between a nucleic acid and a ligand, a method of detecting polymorphism by specific binding between ligand and receptor, focusing in particular on a single kind of single-nucleotide polymorphism (i.e., two kinds of nucleic acids, one containing a mutation site and the other containing the standard site), will be described below.

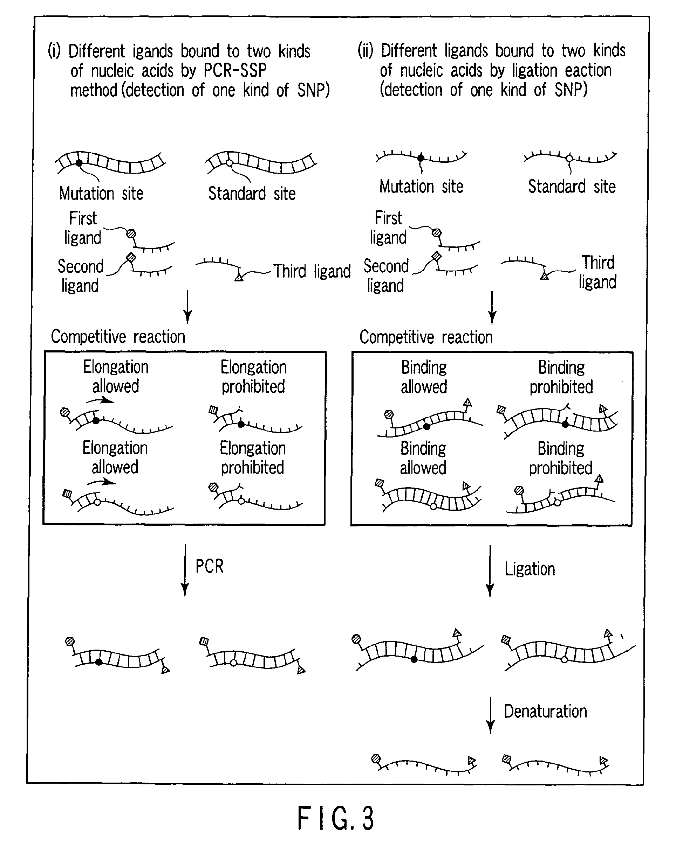

First, a method of binding different ligands respectively to two kinds of nucleic acids (one kind of SNP) by the PCR-SSP method will be described (FIG. 3(i)). The PCR-SSP method (Polymerase Chain Reaction-Sequence Specific Primer method) is a method of controlling amplification by designing the elongation-sided terminal of one of two kinds of primers to overlap with the SNP site of the target nucleic acid and examining the matching between the SNP site and the primer elongation-sided terminal.

As shown in FIG. 3(i), three kinds of primers respectively modified with the first to third ligands are prepared (hereinafter, referred to respectively as first to third primers). The first primer has a sequence corresponding to the nucleotide sequence including a mutation site and its elongation-sided terminal corresponding to the nucleotide of the mutation site. On the other hand, the second primer has a sequence corresponding to the nucleotide sequence including the standard site and its elongation-sided terminal corresponding to the nucleotide of the standard site. The third primer has a nucleotide sequence allowing PCR with the first and second primers. Single-nucleotide polymorphism refers to a state or a site where a nucleotide in a particular gene nucleotide sequence is different. The nucleotide sequences upstream and downstream of the polymorphism are common between the standard and variant nucleotide sequences, and thus the first and second primers have the same nucleotide sequence, except that the elongation-sided terminals are different from each other by a single base. Hybridization under the condition including these two primers leads to competitive binding of the first and second primers to nucleic acids (competitive reaction). However, as shown in the FIG. 3(i), under the competitive reaction condition, the first ligand-modified primer does not cause a polymerase elongation reaction of the standard nucleotide, because the primer has a mismatched elongation-sided terminal. On the other hand, the second ligand-modified primer does not cause a polymerase elongation reaction of the variant nucleotide, because the primer has a mismatched elongation-sided terminal. It is therefore possible to establish a correlation between a nucleic acid and ligands more reliably by hybridization under such a competitive reaction condition.

Then, the ligand-modified nucleic acid is obtained by amplification of the nucleic acid by PCR method (FIG. 3(i)). The nucleotide including the mutation site is obtained as a nucleic acid modified with the first and third ligands, while the nucleotide including the standard site is obtained as a nucleotide modified with the second and third ligands. The reaction, which is a competitive reaction, is extremely high in accuracy.

The method of binding different ligands respectively to two kinds of nucleic acids (one kind of SNP) by ligation reaction will be described below (FIG. 3(ii)). Similarly to the PCR-SSP method above, it is also possible to correlate a nucleotide with a ligand in a ligation reaction. As shown in FIG. 3(ii), three kinds of nucleic acid probes modified respectively with the first to third ligands are prepared (hereinafter, referred to respectively as first to third nucleic acid probes). The first nucleic acid probe has a nucleotide sequence corresponding to the nucleotide sequence including a mutation site and the binding-sided terminal has a nucleotide sequence corresponding to the nucleotide of the mutation site. On the other hand, the second nucleic acid probe has a nucleotide sequence corresponding to the nucleotide sequence including the standard site, and the binding-sided terminal has a nucleotide corresponding to the nucleotide of the standard site. The third nucleic acid probe has a nucleotide sequence allowing ligation reaction with the first and second nucleic acid probes. As described above, the nucleotide sequences upstream and downstream of the single nucleotide polymorphism are the same between standard and variant nucleotides, and the nucleotide sequences of the first and the second nucleic acid probe are different from each other only at the binding-sided terminal and the same in other sequences as each other. Accordingly, the first and second nucleic acid probes bind to nucleic acids competitively (competitive reaction). However, as shown in the figure, under the competitive reaction condition, the first nucleic acid probe cannot react subsequently with the standard nucleotide in ligation reaction, together with the third ligand-modified nucleic acid probe, because the probe has a mismatched binding-sided terminal. On the other hand, the second nucleic acid probe cannot react with the variant nucleotide in ligation reaction, together with the third nucleic acid probe, because the probe has a mismatched binding-sided terminal. Then, the first and third nucleic acid probes are ligated on the variant nucleotide, and the second and third nucleic acid probes are ligated on the standard nucleotide. Subsequent denaturation reaction gives ligand-modified nucleic acids (FIG. 3(ii)). Accordingly, the nucleic acid including the mutation site are obtained as the nucleic acid modified with the first and third ligands, and the nucleic acid including the standard site as the nucleic acid modified with the second and third ligands.

Because the ligation reaction requires no step of amplifying the sample, it is possible to simplify the reaction step and thus reduce cost and shorten reaction period. However, it is disadvantageous in that the sensitivity is lower and that the probe design is allowed only in the regions upstream and downstream of the SNP site. There is a possibility of a similar sequence present in the region different from the target SNP site, depending on the sample, and, in such a case, the method has a disadvantage of deteriorated accuracy. Accordingly, it is possible to solve the problem by performing the PCR reaction in the region close to the target SNP site and amplifying the nucleic acids including the target SNP for improvement in sensitivity and accuracy.

FIGS. 1 to 3 show the structure of the simplest ligands. The primer and the nucleic acid probe preferably have a nucleotide number of 3 to 60. The number and the kinds of the ligands are not limited thereto, and the first to third ligands may contain two or more molecules respectively. It is also possible to detect a nucleic acid, even in the state without the first or second ligand. In addition, it is possible to simplify the reaction system by reducing the number of the ligands.

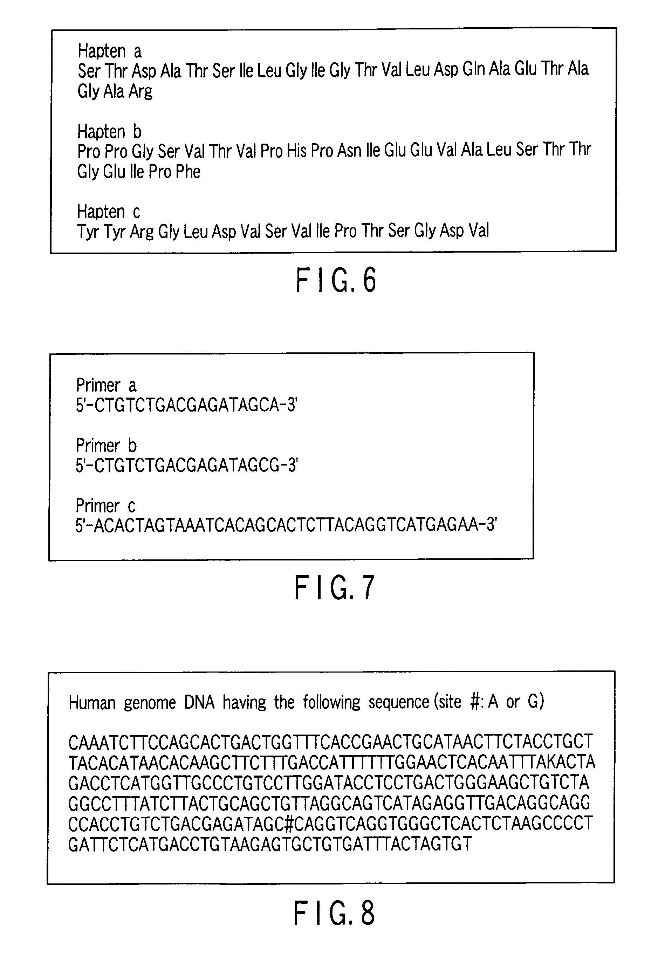

Hereinafter, the embodiments thereof will be described with reference to the specific ligand substance, primer, nucleic acid probe, and target nucleic acid shown in FIGS. 6 to 9.

### (i) PCR-SSP method

Haptens a to c (polypeptides) are used as ligand substances (FIG. 6), and primers a to c respectively modified with haptens a to c are prepared (FIG. 7). Then, the sample shown in FIG. 8 was amplified by the PCR-SSP method. When the sample SNP site (site # in FIG. 8) is present as two types of alleles, nucleotide A (adenine) and nucleotide G (guanine), the allele types include homo A/A, hetero A/G, and homo G/G. When the sample SNP site is homo A/A, the primer a shown in FIG. 7 is matched with the SNP site and amplified together with the primer c by PCR. Because the primer b is not amplified, the amplification products are double-stranded DNAs modified respectively with haptens a and c both at the 5' terminals. Similarly when the sample SNP site is G/G, the PCR products are double-stranded DNAs modified respectively with haptens b and c both at the 5' terminals. Alternatively, when the sample SNP site is A/G, obtained are amplification products including double-stranded DNAs modified respectively with haptens a and c and double-stranded DNAs modified respectively with haptens b and c.

In the method above, for example the amplification may be performed only with primer a and primer c, instead of using three kinds of primers. In such a case, products obtained by amplification with primers b and c in a separate container are mixed, but the method is lower in accuracy.

In the embodiment described above, it is possible in detecting a nucleotide SNP to convert a nucleic acid to a nucleic acid modified with haptens corresponding to the polymorphism.

It is also possible to detect multiple SNPs in the target nucleic acid. In this case, the target nucleic acid may be amplified by the PCR-SSP method, by using primers modified with different haptens according to the SNP site.

### (ii) Ligation reaction

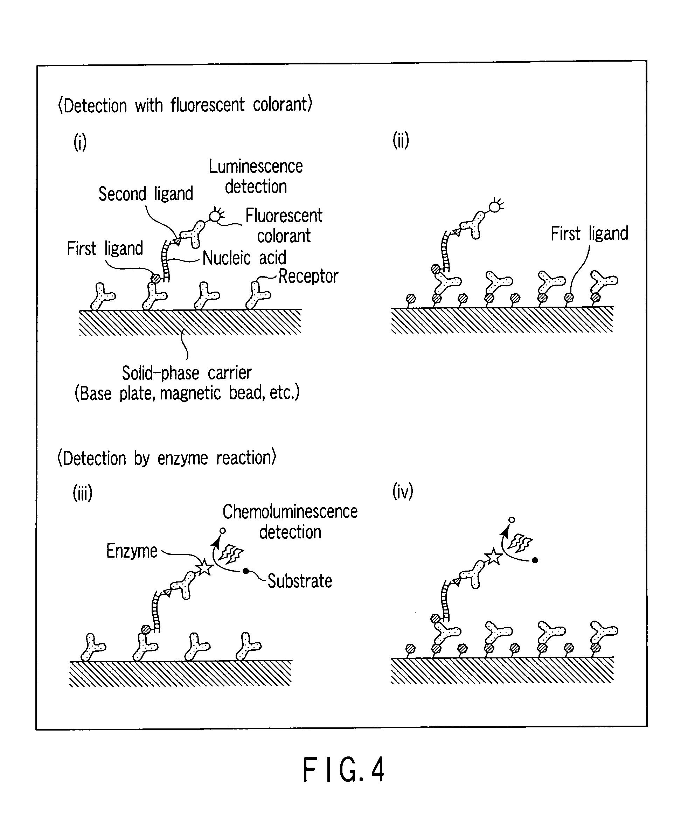

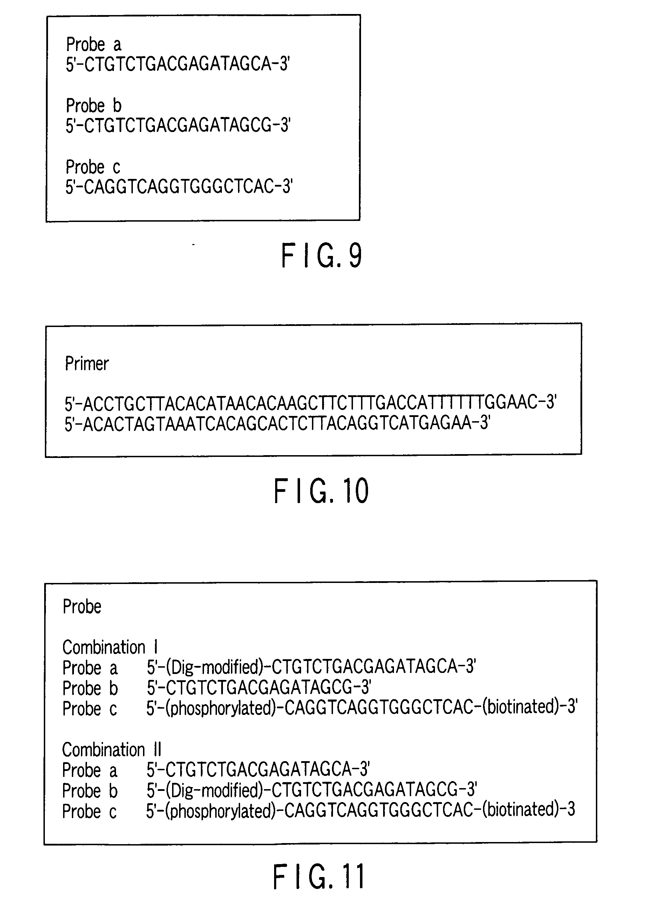

Haptens a to c (polypeptides) are used as ligand substances (FIG. 6), and nucleic acid probes a to c modified respectively with haptens a to c are prepared (FIG. 9). Then, the sample shown in FIG. 8 was ligated. When the sample SNP site is homo A/A, the probe a shown in FIG. 4 is matched with the SNP site and ligated together with the probe c by ligase. The probe b is not ligated. The double-stranded nucleic acids are denatured into single-stranded chains by making the buffer alkaline after reaction, and the reaction product gives single-strand DNAs modified with haptens a and c respectively, both at 5' and 3' terminals. Similarly when the sample SNP site is G/G, the ligation product gives single-strand DNAs modified with haptens b and c respectively at both terminals. Alternatively when the sample SNP site is A/G, ligation product gives a mixture of single-strand DNAs modified with haptens a and c and single-strand DNAs modified with haptens b and c. In the method above, for example, the amplification may be performed only with probes a and c, instead of using three kinds of probes. In such a case, products obtained by amplification with primers b and c in a separate container are mixed, but the method is lower in accuracy.

In the embodiment described above, it is possible in detecting a nucleotide SNP to convert a nucleic acid into that modified with haptens corresponding to the polymorphism.

A method of detecting a single kind of SNP has so far been described, but it is also possible to detect multiple kinds of SNPs in the present invention. For detection of multiple kinds of SNPs, the number of the primers/probes increases according to the kinds of SNP sites, because a competitive reaction is introduced to each SNP. For two kinds of SNPs, six primers/probes are needed, and at least fourth and fifth ligands are also needed. The sixth ligand corresponding to the third ligand may be used, but it is possible to simplify BF separation after reaction and thus to reduce the cost by using the third ligand commonly for detection of respective SNPs. Detection of respective SNPs may be performed in separate containers, or simultaneously in a single container. In particular, when in detecting two or more SNPs in a single container, it is possible to perform a purification step of BF separation in the order independently of the kind of SNP, by using ligands corresponding to the third ligand different for respective SNPs (sixth, ninth, and other ligands). Advantageously, it is possible to perform various measurements simultaneously at low cost, by using the third ligand commonly in detection of two or more SNPs in a single container.

### 2. Step of detecting a ligand-modified nucleic acid

### (1) Detection of only one target nucleic acid

FIG. 4 shows a method of detecting a ligand-modified nucleic acid. There are many methods of detecting a nucleic acid, and the present invention includes any aspect which is applicable to the present invention. A typical aspect among them is a detection method of using a fluorescent colorant. As shown in FIG. 4(i), a receptor binding to the first ligand specifically is immobilized on a solid-phase carrier. A receptor binding to the second ligand specifically is previously modified with a fluorescent colorant. The ligand-modified nucleic acid is bound to the solid-phase carrier via the first ligand. The receptor modified with the fluorescent colorant is then bound to the second ligand. The nucleic acid is then detected, by means of an optical device detecting the fluorescent colorant. As shown in FIG. 4(ii), the first ligand may be immobilized on the solid-phase carrier in advance. In such a case, the immobilized first ligand is first allowed to react with the receptor specifically binding thereto. Then, the first ligand in the ligand-modified nucleic acids is bound to the receptor. The solid-phase carrier supporting the ligand substance is superior in storage stability to the receptor-immobilized solid-phase carrier, which is very advantageous in practical application of the test method.

As shown in FIG. 4(iii), the nucleic acid can also be detected in enzyme reaction. The enzyme for use is, for example, an alkali phosphatase or a peroxidase. In such a case, the receptor binding to the second ligand specifically is previously modified with an enzyme, and chemoluminescence generated by reaction of the enzyme with its substrate is detected. Similarly to the case of using a fluorescent colorant, a solid-phase carrier previously carrying the immobilized first ligand may be used (FIG. 4(iv)).

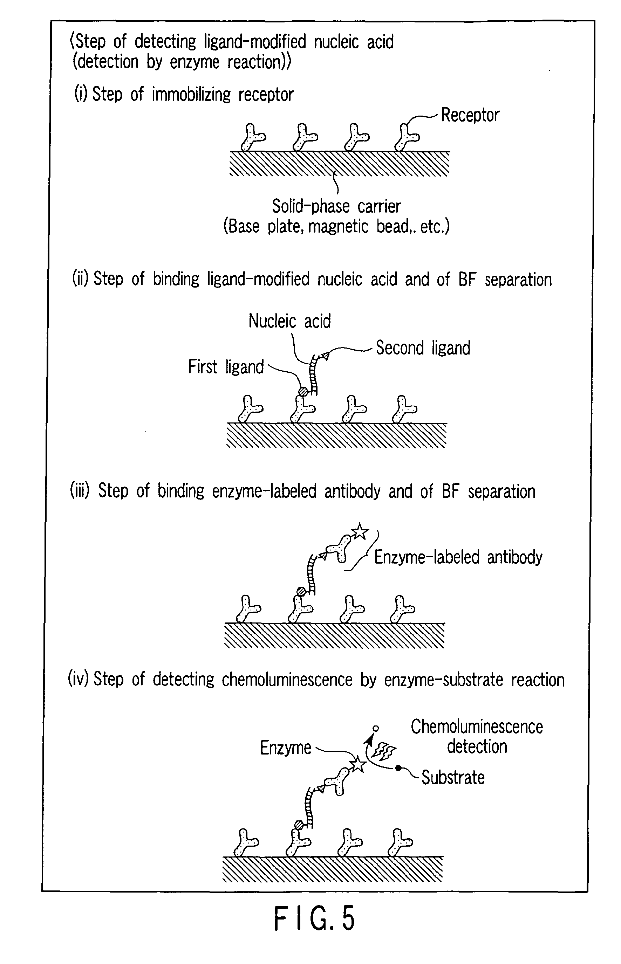

Hereinafter, the detection step in the aspect of FIG. 4(iii), among FIGS. 4(i) to (iv), will be described in more detail (FIG. 5). As shown in FIG. 5(i), a receptor is bound to a solid-phase carrier in advance. Alternatively, a solid-phase carrier supporting a receptor previously immobilized may be used. Then, the ligand-modified target nucleic acid is bound to the solid-phase carrier by specific binding between the receptor and the ligand. Then, the unbound free nucleic acids are removed by BF separation (FIG. 5 (ii)). The enzyme-modified antibody is then bound to the second ligand. The unbound free labeling antibody is then separated by BF separation (FIG. 5 (iii)). Finally, the target nucleic acid is identified, while chemoluminescence by enzyme-substrate reaction is induced and detected (FIG. 5 (iv)). The detection methods shown in FIGS. 4(i), (ii), and (iv) may also be performed in a similar manner.

### (2) Detection of multiple kinds of target nucleic acids

The detection step is the same, even when there are multiple kinds of target nucleic acids. For convenience of description, nucleic acids modified with the first and the second ligands were used in FIGS. 4 and 5. When multiple kinds of nucleic acids are used, in particular when the SNPs therein are to be detected, nucleic acids modified with the first and third ligands and/or nucleic acids modified with the second and third ligands are detected. For example, in the case of the single nucleotide polymorphism described above, variant nucleic acids are modified with the first and third ligands, while the standard nucleic acids are modified with the second and third ligands. Thus, it is possible to detect the variant nucleic acids and the standard nucleic acids, by preparing a solid-phase carrier supporting an immobilized receptor specifically binding to the first ligand and a solid-phase carrier supporting an immobilized receptor binding to the second ligand specifically.

It is also possible to detect the variant nucleotides and the standard nucleotides simultaneously, by using a solid-phase carrier supporting an immobilized receptor specifically binding to the third ligand. Both the variant nucleotides and the standard nucleotides can bind to the solid-phase carrier supporting an immobilized receptor specifically binding to the third ligand. On the nucleic acid-solid-phase carrier, the variant nucleic acid has the first ligand in the unbound state, while the standard nucleic acid has the second ligand in the unbound state. Thus, it is possible to detect the variant nucleic acids and the standard nucleic acids simultaneously, by modifying the receptor binding to the first ligand specifically and the receptor binding to the second ligand specifically respectively with different labeling substances in advance and by using the labeling substances.

It is also possible to detect the nucleic acid, even when there is no first or second ligand. It is possible to simplify the reaction system, by reducing the number of the ligands used.

Hereinafter, the receptor and the solid-phase carrier for use in the present invention will be described.

### (i) Receptor

The term receptor means a substance that binds to a specific ligand substance. For example, avidin is the receptor when a biotin is selected as the ligand, and an anti-hapten antibody is the receptor when a hapten is selected as the ligand. The anti-hapten antibody is not particularly limited, but is desirably an antibody after affinity purification or a monoclonal antibody, for improvement in specificity and detection accuracy. The ligand and the receptor may be respectively an antigen and an antibody, and the specific binding between ligand and receptor may be formed by antigen-antibody reaction. Detection of nucleic acid by immune reaction allows high-reactivity and high-accuracy detection.

### (ii) Solid-phase carrier

### i) Plate-shaped carrier

The term solid-phase carrier means any carrier allowing immobilization of the receptor above. The material and the shape of the carrier are not particularly limited. Examples of the materials include glass, silicon, metals, metal oxides, resins, rubbers, ceramics, and the like. For reaction with a linker, the carrier surface preferably has active groups such as hydroxyl, carboxyl, amino, and thiol groups. Alternatively, a gold surface, which forms a coordination bond with a thiol group, may be used without the active groups. The shape is preferably the same as a slide glass (approximately 25×75×1 mm) for use in a DNA microarray scanner allowing high-sensitivity fluorescence measurement, or a microplate (approximately 86×128 mm) for use in a chemoluminescent plate reader. The carrier may be permeable or nonpermeable to liquid. Such a liquid-permeable carrier is in many cases made of a porous material, and thus has a larger surface area, advantageously leading to increase in the amount of the antibody immobilized and in the freedom of the liquid-traveling direction, and thus to easier automation of the reaction. However, it also leads to disadvantages such as restriction on the choice of the material and necessity for occasional modification of the apparatus, and thus the carrier should be selected carefully according to its application.

For example, a series of reactions carried out thereon are shown below. Reactions other than those described below may be used in the present invention, if they satisfy the requirements described above.

The carrier for use is a low-fluorescence silica glass. The glass is degreased well with alcohol and washed with purified water. A solution of 3-wt% aminopropyltrimethoxysilane (manufactured by Shin-Etsu Chemical Co., Ltd.) in ethanol is then prepared. The glass above is immersed in the solution at room temperature, allowed to react for 1 hour while stirred, cleaned with ethanol and additionally with purified water, and dried at 130°C for 20 minutes.

Subsequently, EDC (manufactured by PIERCE) was dissolved in a MES buffer to a concentration of 5 wt%, and the treated glass is immersed in the solution at room temperature and allowed to react for 1 hour while the solution is stirred. After reaction, the glass was washed with the MES buffer and then with purified water and dried at room temperature by the spin-dry method.

The anti-hapten antibody solutions are solutions respectively containing anti-hapten "a" antibody and anti-hapten "b" antibody respectively corresponding to haptens a and b shown in FIG. 6 that are affinity-purified and dissolved in a MES buffer to a concentration of 5 mg/ml, and the solutions were spotted at different positions of the glass plate. The solution is spotted by a method of using a needle-type spotting device SPBIO (manufactured by Hitachi Software). Each solution was spotted on four positions of the glass, allowed to react at room temperature for 1 hour, and then, the glass was immersed in a PBS buffer containing BSA (bovine serum albumin) at a concentration of 0.1 wt% at room temperature for 1 hour. After immersion, the glass plate is washed with a PBS buffer and a PBS buffer at 1/10 concentration in that order, dried by spin-dry method, and stored in the dry state in a cold dark place.

The carrier can be prepared as described above. It is also possible to increase the kinds of the target nucleic acids further, by immobilizing more than two kinds of anti-hapten antibodies.

### ii) Particulate carrier

In another aspect of the present invention, a particulate carrier may be used as the solid-phase carrier and the receptor may be immobilized thereon.

A particulate carrier of a resin is used frequently. Typical materials include natural rubbers, styrene and styrene copolymers, polyurethane, acrylic resins, and the like, but the material for the particulate carrier for use in the present invention is not particularly limited. It is also possible to add a magnetic substance to the particulate carrier. Addition of a magnetic substance to the particulate carrier makes it easier to handle the particulate carrier.

The method of immobilizing the receptor on a particulate carrier may be physical adsorption, chemical adsorption, or the like. Physical adsorption is used frequently because the step is simpler, but chemical adsorption is also favorable for preservation of the activity of the anti-hapten antibody and for stabilized reaction. Mostly in chemical adsorption, the carrier and the antibody are bound to each other covalently via a chemical substance called a linker. An optimal linker is selected according to the material and the surface state of the carrier and the kind of the antibody.

For applications where biotin is used as the ligand and avidin or streptoavidin as the receptor, magnetic beads carrying immobilized avidin or streptoavidin are available commercially and may be used.

After preparation of the receptor-immobilized solid-phase carrier, a nucleic acid-solid-phase carrier complex is obtained by binding at least one of the ligand-modified nucleic acids to the solid-phase carrier via a ligand.

### (3) Embodiments of the invention

### (i) Plate-shaped carrier

Hereinafter, the step of detecting a nucleic acid by using a nucleic acid containing the single nucleotide polymorphism shown in FIG. 8 will be described. Similarly to the PCR-SSP method and the ligation reaction described above, the haptens, primers, and probes used are shown respectively in FIGS. 6, 7 and 9.

The hapten-modified nucleic acid is dissolved in a MES buffer; the solution is spotted on a substrate and allowed to react at 37°C for 2 hours; and the resulting substrate is washed with the MES buffer. Then, if a nucleic acid modified with hapten a is present in the solution spotted on the substrate, the nucleic acid binds to the anti-hapten a antibody. Similarly, if a nucleic acid modified with hapten b is present in the solution, the nucleic acid binds to the anti-hapten b antibody. As a result, if the SNP site in the target nucleic acid is A/A, there is unbound hapten c present on the spot of the carrier having an immobilized anti-hapten a antibody. There is no hapten c present on the spot of the carrier having an immobilized anti-hapten b antibody. Similarly, if the SNP site of the target nucleic acid is G/G, there is unbound hapten c present on the spot of the carrier having an immobilized anti-hapten b antibody. There is no hapten c present on the spot of the carrier having an immobilized anti-hapten a antibody. Further if the target nucleic acid SNP site is A/G, there is unbound hapten c present on the spots of the carrier having an immobilized anti-hapten a antibody and an anti-hapten b antibody.

A solution of an anti-hapten c antibody labeled with a fluorescent colorant CY5 in a MES buffer at a concentration of 10 µ/ml is spotted on the carrier after reaction, and is allowed to react at 37°C for 2 hours. Then, the resulting carrier is washed with the MES buffer and then with a MES buffer at a 1/10 concentration, and subsequently, spin-dried, to give a carrier having CY5 present only on the spot where there is hapten c.

The carrier is placed in GenePix4000B (manufactured by AXON Instruments), and the luminescence at an excitation wavelength of 635 nm and a detection wavelength range of 650 nm to 690 nm is determined. It is possible to detect the target nucleic acid SNP, by analyzing the fluorescence intensity in the scanned image and the information on the position of the spots on the carrier.

Alternatively, an anti-hapten c antibody labeled with an enzyme, for example with alkali phosphatase, may be used instead of the anti-hapten c antibody labeled with the CY5 above. In such a case, hapten c on the carrier and the anti-hapten c antibody labeled with the alkali phosphatase are bound to each other in reactions similar to those above. After washing, a solution of the substrate (e.g., CDP-Star (manufactured by Calbio. Chem.)) is spotted, and the chemoluminescence intensity is determined in a Luminescencer (manufactured by ATTO) for detection of the target nucleic acid SNP.

Alternatively, hapten a or b may be immobilized on the carrier, replacing the anti-hapten antibody. Then, in the immobilization method, the processing condition for example for the linker, may be exactly the same if the C-terminal carboxyl group of hapten is used. A mixed solution containing anti-hapten a antibody and anti-hapten b antibody is spotted on the hapten-immobilized carrier prepared. The solution is allowed to react at 37°C for 2 hours and then washed similarly to the method above. The anti-hapten a antibody and the anti-hapten b antibody bind respectively to the immobilized haptens a and b, and subsequently, the luminescence therefrom can be detected similarly.

### (ii) Particulate carrier

Hereinafter, the step of detecting a nucleic acid by using a magnetic particulate carrier will be described.

An anti-hapten c antibody is immobilized on a magnetic particulate carrier, and a nucleic acid is detected by using the resulting carrier. The particulate carrier supporting the immobilized anti-hapten c antibody is dispersed in a phosphate buffer; and the nucleic acid modified with the hapten corresponding to the SNP above is mixed with the dispersion, allowing the nucleic acid to bind to the particulate carrier. The particulate carrier, which is magnetic, can be relocated by external application of magnetic force, and thus the particles can be collected at a particular position in the solution. For example, the particulate carrier is temporarily immobilized on the container wall by using a magnet. The solution is removed while the particulate carrier is immobilized on the container wall, and a new phosphate buffer is added. The particulate carrier is then redispersed in the new phosphate buffer solution, after the magnet is removed. It is possible to remove unreacted nucleic acids and by-products in the solution by repeating the steps several times (BF separation step). After the BF separation step, the solution containing the particulate carrier uniformly dispersed in the phosphate buffer is divided into two portions. The number of the portions is determined according to the numbers of the analyte nucleic acids and the haptens used. The target nucleic acid binds to the particulate carrier via hapten c, leaving hapten a in the unbound state in the case of A/A, hapten b in the case of G/G, and haptens a and b in the case of A/G. Thus, it is possible to detect the target nucleic acid by using the labeled anti-hapten a and b antibodies.

An enzyme-labeled anti-hapten a antibody and an enzyme-labeled anti-hapten b antibody are added separately to each of the two portions of the solution containing the particulate carrier and allowed to react therein. After reaction, the unreacted enzyme-labeled anti-hapten antibodies are removed by repeating the BF separation step once again. Then, alkali phosphatase or peroxidase, for example, is used favorably as the enzyme, but the enzyme is not limited thereto.

Finally, it is possible to detect target nucleic acid SNPs simultaneously by adding enzyme substrate compounds respectively to the containers, allowing reactions for a certain period of time, and then, measuring, for example, the light intensity or the change in absorbance.

It is possible to detect the target nucleic acid SNPs simultaneously by fluorescence intensity by labeling a fluorescent colorant on the anti-hapten a and b antibodies replacing an enzyme.

It is also possible to detect the nucleic acid by using a carrier supporting an immobilized anti-hapten a antibody carrier and a carrier supporting an immobilized anti-hapten b antibody. Anti-hapten a antibody and anti-hapten b antibody are used as the antibodies to be immobilized on a magnetic particulate carrier, and particulate carriers with respective antibodies are prepared and placed in separate containers. The nucleic acid modified with the hapten corresponding to the target nucleotide SNP is added in two portions respectively to the separate containers and allowed to react therein. An enzyme-labeled anti-hapten c antibody is added thereto and allowed to react after BF separation. After the reaction and additional BF separation step, an enzyme substrate compound is added finally to the respective containers and allowed to react for a certain period of time, and the target nucleic acid SNP is detected by measurement of the light intensity, change in absorbance change, or the like. For the reasons described above, the number of the antibodies immobilized on the particulate carrier is determined according to the numbers of the target nucleic acids and haptens used. In addition, the kinds of the enzyme and the fluorescent colorant can also be modified.

The BF separation step may be carried out by using a non-magnetic particulate carrier. Examples of the other methods include: (i) separation of the particulate carrier by filtration, (ii) precipitation of the particulate carrier and removal of the supernatant, for example, by still standing or centrifugation, and (iii) direct solution exchange, when a particulate carrier having a large particle diameter cannot be pipetted.

Nucleic acid SNPs are determined in all of the embodiments above, but the present invention is easily applicable to determination of other mutations and also of the expression amount of a particular nucleic acid. Such analysis can be performed in a completely similar manner, except that the primer and the probe are designed properly according to the sequence of the desirable nucleic acid the mutation and the expression amount of which are analyzed.

### [Example]

A nucleic acid having the single-nucleotide polymorphism shown in FIG. 8 was detected by ligation reaction. Before the ligation reaction, the nucleotide sequences upstream and downstream of the single-nucleotide polymorphism were amplified by PCR for removal of pseudo-sequences and improvement of detection sensitivity. The carrier used was magnetic particles, and the nucleic acid was detected by an enzyme substrate reaction.

### 1. Preparation of reagents

The following reagents were prepared.
- Sample: combinations of human genome DNAs having the sequences shown in FIG. 8
   Combination i: combination of allele A (base at the site # in FIG. 8: A) and allele A (A/A)
   Combination ii: combination of alleles A and G (A/G)
   Combination iii: combination of alleles G and G (G/G)
- PCR primer: PCR primer having the sequence shown in FIG. 10
- PCR reagent: "Accuprime Super Mix II" manufactured by Invitrogen, catalogue No. 12341-012
- Probe: combination of the probes shown in FIG. 11.
   Combination I: Among the probes having the sequences shown in FIG. 9, probe a with its 5' terminal modified with digoxigenin (Dig), probe b with its 5' terminal unmodified, and probe C with its 3' terminal modified with biotin and its 5' terminal phosphorylated.

   Combination II: Among the probes having the sequence shown in FIG. 9, probe a with its 5' terminal unmodified, probe b with its 5' terminal modified with digoxigenin (Dig), and probe c with its 3' terminal modified with biotin and with its 5' terminal phosphorylated.
- Ligase: "Taq DNA Ligase" manufactured by BioLabs, catalogue No. M0208S
- Magnetic particulate carrier: "Dynabeads M-280 streptavidin" manufactured by DYNAL, catalogue No. 112.06
- Enzyme-labeled antibody: "Anti-Digoxigenin (rabbit polyclonal antibody with ALP) manufactured by DAKO, catalogue No. D5105
- Luminescence substrate: "CDP-Star substrate" manufactured by Wako, catalogue No. 69086

### 2. Reaction

The reaction was performed according to the following procedure, by using each of the three sample combinations and the reagents above.
- Sample previously adjusted to 5 ng/µl: 1 µl
- PCR reagent: 10 µl
- Each primer adjusted to 1 µM: 1 µl (total of 2 µl)
- Purified water: 7 µl
   Total: 20 µl

The PCR solution thus adjusted was subjected to the following reaction in a thermal cycler.
(1) 94°C for 2 minutes
(2) 94°C for 30 seconds
(3) 68°C for 6 minutes
(4) The operations (2) and (3) are repeated 40 times.

After PCR, the amount of the PCR product was determined by absorbance measurement, and the solution is diluted to a concentration of 4 ng/µl.

The diluted sample is divided into two portions, and each portion is mixed with a
- Sample previously adjusted to 4 ng/µl: 1 µl
- Taq Ligase buffer (attached to kit): 3 µl
- 40 U/µl Taq Ligase: 0.5 µl
- Each probe combination I or II adjusted to 10 nM: 1 µl (total 3 µl)
- Purified water: 22.5 µl
   Total: 30 µl, and
   the mixture was subjected to the following reaction in an incubator.
   (1) 95°C for 5 minutes
   (2) 58°C for 15 minutes

After reaction, 5 µl of a magnetic particulate carrier is added thereto, and the mixture is allowed to react at room temperature for 5 minutes.

After reaction, the magnetic carrier is subjected to BF separation reaction twice with a phosphate buffer.

20 µl of the 100-time diluted enzyme-labeled antibody solution is added to each of them, and the mixture is allowed to react at room temperature for 60 minutes.

The magnetic carrier was subjected to BF separation reaction respectively, three times with the phosphate buffer.

100 µl of the substrate is added respectively, and the mixture is allowed to react at room temperature for 1 minute.

The mixtures are transferred onto a 96-well black plate for measurement, and the luminescence from each of the well is determined as integrated for 10 seconds in a luminescence analyzer (Luminescencer-JNRII, manufactured by ATTO).

### 3. Measurement results

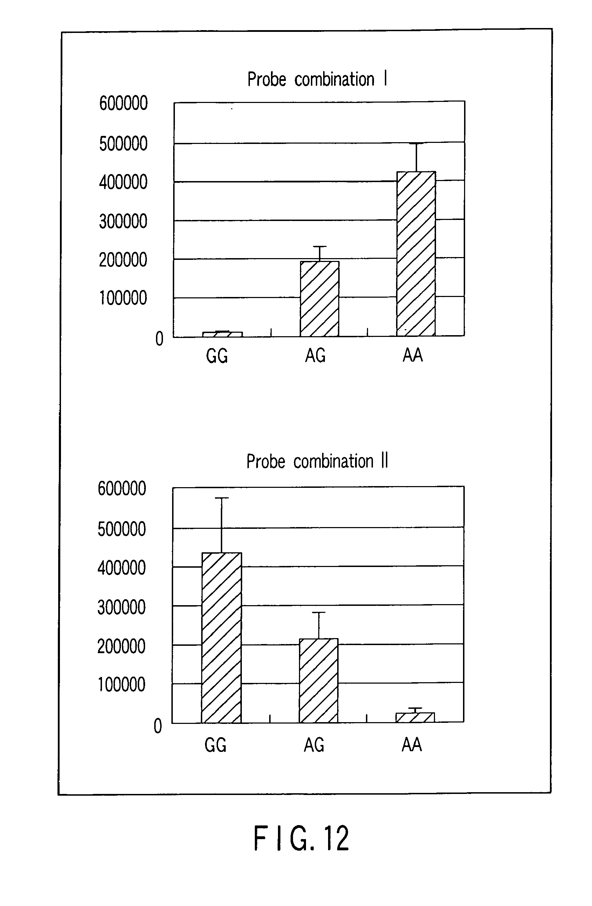

The reaction is repeated nine times for each sample, and the results are summarized in FIG. 12. FIG. 12 is a graph showing the average luminescence intensity in each combination of sample and probe, indicating that the luminescence intensity corresponding to the sample SNP is obtained. In particular, sample combinations of A/A, A/G and G/G are differentiated definitely, indicating that the present invention is very useful in differentiating, using merely one reaction, A/A from A/G and G/G from A/G, which was difficult by traditional methods.

### [Modification]

Hereinafter, a modification will be described. In the present modification, the nucleic acid was amplified not by the PCR method but by the LAMP (Loop Mediated Amplification) method, and the nucleic acid containing a single-nucleotide polymorphism was detected.

In reaction and measurement by the LAMP method, used are the following reagents:

In 100 µL mixture:
20 mM Tris-HCl pH 8.8
10 mM KCl
10 mM (NH₄)₂SO₄
4 mM MgSO₄
1M betaine
0.4 mM dNTP
8U Bst DNA polymerase

The primer having the following sequence was placed in the reaction solution, to give a reaction solution. Inner primers FA(G) and RA(G) were the primers for detection of allele G in the sample, and the primers were labeled with biotin at the 5' terminal. Also, inner primers FA(A) and RA(A) were primers for detection of allele A, and the primers were labeled with digoxigenin (Dig) at the 5' terminal.
Primer:
1600 nM inner primer FA(G)
biotin-CTCCCCTCAGAACATAACATAGTAATGCGGTAAGTCGCATAAAAACCATTC
1600 nM inner primer FA(A)
Dig-TTCCCCTCAGAACATAACATAGTAATGCGGTAAGTCGCATAAAAACCATTC 1600 nM inner primer RA(G)
biotin-GTGAAAATTCCCCTAATTCGATGAGGTCGGCGCATAGCTGATAACAAT 1600 nM inner primer RA(A)
Dig-TTGAAAATTCCCCTAATTCGATGAGGTCGGCGCATAGCTGATAACAAT 400 nM outer primer F3
GCTTATCTTTCCCTTTATTTTTGC
400 nM outer primer R3
GCTGATCGGCAAGGTGTTCT

These primers were prepared on ice for prevention of undesirable reaction during preparation of the reaction solution.

λDNA (SEQ ID No. 1) of 1×172 bases was used as the polynucleotide to be amplified (template nucleic acid) without thermal denaturation.

<SEQ ID No. 1>
GCTTATCTTTCCCTTTATTTTTGCTGCGGTAAGTCGCATAAAAACCATTCTTCAT
AATTCAATCCATTTACTATGTTATGTTCTGAGGGGA#TGAAAATTCCCCTAATTC
GATGAAGATTCTTGCTCAATTGTTATCAGCTATGCGCCGACCAGAACACCTTGCC
GATCAGC

The 92nd nucleotide # in the nucleotide sequence of SEQ ID No. 1 is the site of single-nucleotide polymorphism, and is G or A. The regions corresponding to the target regions F1c, F2c, F3c, R1, R2, and R3 are the following:

Target region (single-nucleotide polymorphism region): only 92nd G or A in the nucleotide sequence of SEQ ID No. 1
F1c: 68th to 91st bases (24 bp) in the nucleotide sequence of SEQ ID No. 1
F2c: 25th to 50th bases (26 bp) in the nucleotide sequence of SEQ ID No. 1
F3c: first to 24th bases (24 bp) in the nucleotide sequence of SEQ ID No. 1
R1: 93rd to 115th bases (23 bp) in the nucleotide sequence of SEQ ID No. 1
R2: 129th to the 152nd bases (24 bp) in the nucleotide sequence of SEQ ID No. 1
R3: 153rd to the 172nd bases (20 bp) in the nucleotide sequence of SEQ ID No. 1

A sample without a template nucleic acid was used as the negative control.

A total of eight samples: two samples with a sequence having G as the 92nd nucleotide in the nucleotide sequence of SEQ ID No. 1 (sample number: 1 to 2, allele combination: G/G), two samples with a sequence having A as the 92nd nucleotide in the nucleotide sequence of SEQ ID No. 1 (sample number: 3 to 4, allele combination: A/A), two samples with a sequence having G or A as the 92nd nucleotide in the nucleotide sequence of SEQ ID No. 1 (sample number: 5 to 6, allele combination: A/G), and two samples of negative control without a template (sample number: 7 to 8) were prepared.

Each template nucleic acid of sample number 1 to 8 was added to the reaction solution in a sample tube; the sample tube was placed on a heat block previously adjusted to 65°C; and each tube was allowed to react, correctly for 45 minutes, before it was removed from the heat block.

The following detection reagents were prepared for detection.
- Antibody-immobilized magnetic particles
   Anti-biotin antibody-labeled magnetic particles ("Dynabeads M-450 Epoxy" manufactured by DYNAL, labeled with "Monoclonal Mouse Anti-Biotin Code No. M0743" manufactured by DAKO),
   Anti-digoxigenin antibody-labeled magnetic particles ("Dynabeads M-450 Epoxy" manufactured by DYNAL, labeled with "Anti-Digoxigenin Unlabeled" manufactured by COVALAB R&D)
- Enzyme-labeled antibody
   Alkali phosphatase-labeled anti-biotin antibody ("Polyclonal Rabbit Anti-Biotin D5107", manufactured by DAKO)
   Alkali phosphatase-labeled anti-digoxigenin antibody ("Polyclonal Rabbit Anti-DIG/AP Rabbit F (ab'), Code No. D5105" manufactured by DAKO)
   After LAMP reaction, the LAMP product was divided into two portions, and subjected to the following reaction.
- 5 µL of the anti-biotin antibody-labeled magnetic particles and the anti-digoxigenin antibody-labeled magnetic particles were added to each portion, and the mixtures were allowed to react at room temperature respectively for 5 minutes.
- After reaction, the particles were treated with a phosphate buffer twice for BF separation reaction.
- 20 µL respectively of the corresponding 100-times-diluted alkali phosphatase-labeled anti-biotin antibody and alkali phosphatase-labeled anti-digoxigenin antibody were added to each portion of the solution, and the mixture was allowed to react at room temperature for 60 minutes.
- The particles were treated with a phosphate buffer respectively thrice for BF separation reaction.
- 100 µL of the substrate was added to each portion, and the mixture was allowed to react at room temperature for 1 minute. "CDP-Star substrate" catalogue No. 69086, manufactured by Wako was then used as the luminescence substrate.
- The sample was transferred onto a 96-well black plate for measurement, and the integral luminescence intensity for 10 seconds is determined in a luminescence analyzer (Luminescencer-JNRII, manufactured by ATTO).

### Measurement results

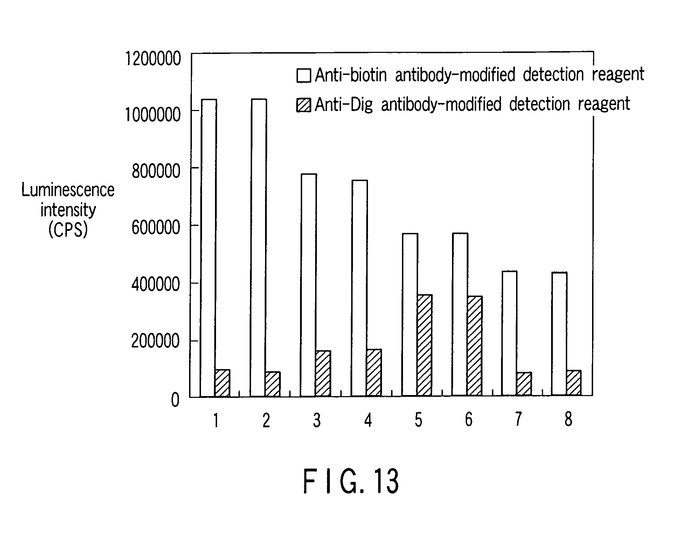

The results of the reactions above are summarized in Table 1 and FIG. 13. Table 1 is a table showing the luminescence intensity (unit: cps) determined in combination of each sample and detection reagents, and FIG. 13 is the graph thereof. The results show that a luminescence intensity corresponding to the sample SNP is obtained. In particular, sample combinations of A/A, A/G and G/G are differentiated clearly, indicating that the present invention is very useful in differentiating, using merely one reaction, A/A from A/G and G/G from A/G, which was difficult by traditional methods, even in amplification methods other than PCR method.

The ligand for use in the present modification is not particularly limited, and may be replaced with any ligand if there is a receptor specifically binding to the ligand. It is also possible to detect the nucleic acid even when ligands are not connected to both of the inner primer FA(G) and the inner primer RA(G) and to both of the inner primer FA(A) and inner primer RA(A), and, for example, biotin may be bound only to the primer 5' terminal of inner primer FA(G) and digoxigenin (Dig) only to the primer 5' terminal of inner primer FA(A).

### [Table 1]

**Table 1**

| Sample No. | Allele type | Anti-biotin antibody-modified detection reagent | Anti-Dig antibody-modified detection reagent |
|---|---|---|---|
| 1 | G/G | 1047721 | 88771 |
| 2 | G/G | 1049072 | 80365 |
| 3 | A/A | 778620 | 156982 |
| 4 | A/A | 759002 | 157842 |
| 5 | A/G | 570613 | 349345 |
| 6 | A/G | 568269 | 345009 |
| 7 | Negative | 428835 | 72617 |
| 8 | Negative | 425009 | 80345 |

II. Method for detecting a nucleic acid by specific binding between ligand and receptor according to the present invention, in particular, the method for detecting a nucleic acid by using coagulation reaction of dispersible particles

The present invention provides the method for detecting a nucleic acid described above, and in particular, a method for detecting a nucleic acid, which is simpler, more accurate and highly reproducible, by using the coagulation reaction of dispersible particles.

In the method for detecting a nucleic acid according to the present invention described above, the target nucleic acid is captured on an immobilization plate, and the captured nucleic acid is detected, for example, by an enzyme substrate reaction.

In contrast, in the method for detecting a nucleic acid by using coagulation reaction of dispersible particles, no immobilization plate is used, but dispersible particles are used instead. Multiple receptors are bound to the surface of the dispersible particles, and the target nucleic acid binds to the dispersible particles via the receptor. The dispersible particles are connected to each other via the target nucleic acid, forming larger aggregates. The aggregation is determined with a spectrophotometer. There is no need for modification of the receptor previously with an enzyme or addition of a substrate to the enzyme in this method, and it is possible to detect a nucleic acid reliably and rapidly by monitoring a physical phenomenon, i.e., aggregation of the dispersible particles. Hereinafter, the method will be described in detail.

The present invention provides a method for detecting a target nucleic acid based on an aggregate generated by crosslinking reaction between the target nucleic acid to be detected and dispersive fine particles, comprising a step of reacting the target nucleic acid to which different kinds of first and second ligands have been attached, with first particles to which two or more receptors specifically binding to the first ligand have been attached and second particles to which two or more receptors specifically binding to the second ligand have been attached, and a step of obtaining the aggregate in such a manner that the first and second particles are mutually linked when the target nucleic acid is bound to the first and second particles, and a large number of the particles are mutually linked when two or more target nucleic acids are bound to one of the particles, and simultaneously bound to the other particles respectively, and measuring the aggregate by spectrophotometry.

The present invention also provides a quantitative method for determining the amount of a nucleic acid, comprising a step of reacting the target nucleic acid whose concentration is known, to which two or more the first and second ligands are bound respectively, to the first and second particles, and measuring the aggregate obtained by the reaction by spectrophotometry, a step of performing similarly the above step about the target nucleic acid of concentration which is different from the above step, and creating a calibration curve from the result of the measurements thereof, a step of reacting the target nucleic acid for detection to the first and second particles, and measuring the aggregate obtained by the reaction by spectrophotometry, and a step of determining the concentration of the target nucleic acid for detection on the basis of the calibration curve.

According to the present invention, it is possible to detect the nucleic acid easily and accurately, based on aggregation of the fine particles, by binding ligands to a nucleic acid and allowing them to react with receptor-bound dispersive fine particles.

The method for detecting a nucleic acid according to the present invention is a method for detecting a nucleic acid by using dispersive fine particles and measuring aggregation of the fine particles. Hereinafter, the method according to the present invention will be described step by step.

First, a ligand is bound to a target nucleic acid to be detected. The target nucleic acid may be a nucleic acid contained in a biological sample or in any sample, but is not limited thereto. The nucleic acids include all nucleic acids and the derivatives thereof such as cDNA, genome DNA, synthetic DNA, mRNA, total RNA, hnRNA, and synthetic RNA, and may be natural or synthetically prepared.

Examples of the ligands bound to the target nucleic acid include, but are not limited to, hydrophilic organic compounds, dioxygen, fluorescein, Alexa, polypeptides having six or more amino acid residues, carbohydrates having two or more saccharides, biotin, proteins, polyhistidine, HA, GST, Flags (e.g., peptide DYKDDDDK), and the like. In particular, biotin, dioxygen, fluorescein, Alexa, and others are commercially available and inexpensive. Carbohydrates and peptides are wider in the freedom in designing the chemical structure and thus multiple kinds thereof are obtained easily. The ligand used may be altered properly, for example, according to the target nucleic acid or the sample to be detected.

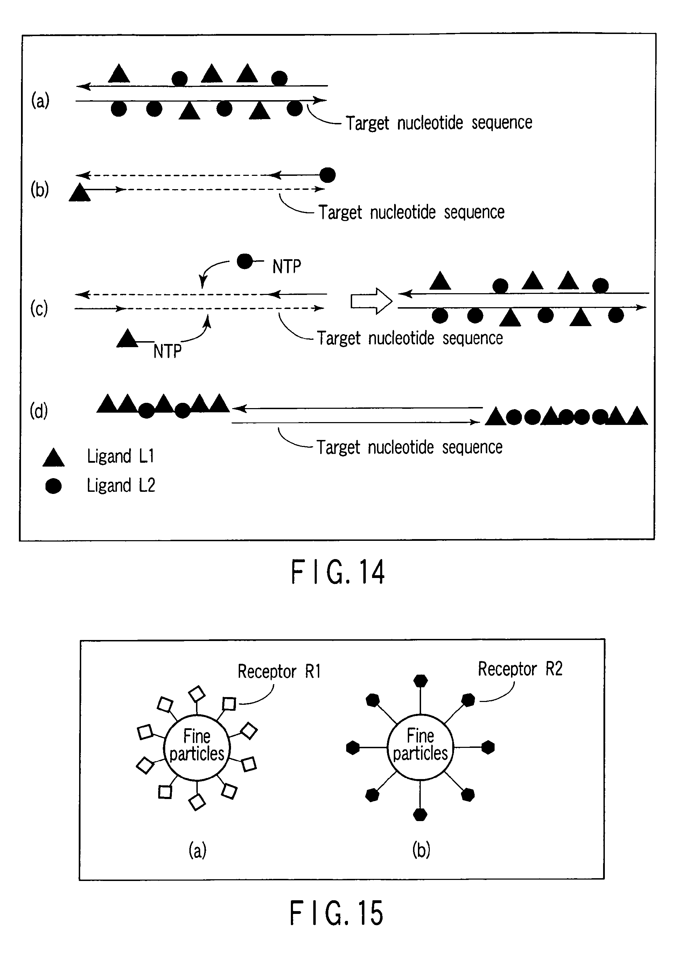

One or more first ligands and one or more second ligands different in kind are bound respectively to the target nucleic acid. The methods of binding the ligand to the target nucleic acid include (i) a method of forming a covalent bond to the base inside or at the terminal of the nucleic acid by using light, platinum or a linker (FIG. 14a), for example, a method of forming a covalent bond between the 5'-terminal amino group of the nucleic acid and the carboxyl group of the ligand, (ii) a method of allowing chain elongation by polymerase using a ligand-bound primer during preparation of nucleic acid (FIG. 14b), (iii) a method of allowing chain elongation by incorporation of a ligand-bound nucleotide during preparation of nucleic acid (FIG. 14c), (iv) a method of tailing a ligand-bound nucleotide to the 3'-terminal of the nucleic acid by using a terminal deoxynucleotidyl transferase (FIG. 14d), and the like.

The method (ii) or (iii) can be used for samples containing a target nucleic acid as well as other nucleic acids, such as biological or any other samples. Any method may be used when only a single target nucleic acid to be detected is present. The target nucleic acid contained, in a biological sample etc. may be amplified by the PCR method before detection or may be used as it is for detection.

As shown in FIG. 14, two kinds of ligands are used as the ligands bound to the target nucleic acid. The nucleic acid structure is flexible, and the nucleic acid should be connected to two kinds of fine particles in the present method, as will be described below, and thus use of two kinds of ligands is desirable.

Each of the two kinds of ligands bound to the target nucleic acid may be two or more in number. The volume ratio of "particle" to "nucleic acid" is extremely large, and thus there is a low possibility of three of more particles binding to one nucleic acid. Accordingly, even if multiple ligands are bound to one nucleic acid, there is seemingly no influence on the aggregation degree of the particles.

Then, a receptor specifically binding to the target nucleic acid-bound ligand is connected to the dispersive fine particles. The dispersive fine particles for use in the present invention refer to particles dispersible in solution, such as colloidal particles, and latex particles are used favorably, but the particles are not limited thereto. The dispersive fine particles are preferably those having a particle diameter of approximately 80 to 300 nm, for measurement of turbidity in an immunonephelometric device.

The latex particles are mainly made of polystyrene and contain additionally copolymerized methacrylic acid for improvement in hydrophilicity and dispersibility. The latex particles include plain-type particles having no functional group on the surface and also functional group-type particles having a functional group. The surface charge on the plain-type particles is negative because of the methacrylic acid present thereon, and thus the particles bind to the positively charged regions of a protein ionically. It is also possible to make the particles form a hydrophobic bond. The plain-type latex particle adsorbs proteins when mixed, and thus is advantageous for immobilization of proteins.

The latex particles having functional groups are designed to have, for example, carboxyl groups or amino groups exposed on the surface, and latex particles having various types of functional groups are available. Methods of binding a receptor to the latex particles include an EDAC method of binding a carboxylic acid group to an amino group with a water-soluble carbodiimide, a method of binding a carboxylic acid group to an amino group by mixing EDC and NHS previously, a method of crosslinking amino groups by using a dipolar linker, a method of binding proteins with an activated aldehyde group or a tosyl group, and the like. Any existing method may be used in the invention, but the EDAC method is particularly desirable. Fine particles other than latex particles may be used for binding a receptor by a known method.

The receptor to be bound to the dispersive fine particles is a receptor specifically binding to the ligand bound to the target nucleic acid to be detected. Examples of the receptors include, but are not limited to, antibodies, lectin, streptoavidin, Ni, and the like. Proteins, in particular antibodies, are used favorably. The method of binding a receptor to the fine particles is selected properly from well-known methods according to the fine particles used.

In an aspect, a single kind of receptor is bound to one kind of dispersive fine particles. In the present aspect, at least two kinds of ligands are bound to the target nucleic acid, and thus at least two kinds of dispersive fine particles are prepared for one kind of target nucleic acid. Specifically, first dispersive fine particles carrying the immobilized receptors corresponding to the first ligand (FIG. 15a) and second dispersive fine particles carrying the immobilized receptors corresponding to the second ligand (FIG. 15b) are prepared.

In another aspect, two or more kinds of receptors may be bound to one kind of dispersive fine particles. In the present aspect, the receptor corresponding to one of two kinds of ligands bound to one target nucleic acid and the receptor corresponding to the other ligand are allowed to bind. Thus, it is designed such that both of the two kinds of ligands for the target nucleic acid are not bound to the same fine particles.

Although the binding of the receptor to the dispersive fine particles in the present step may be performed during detection of the nucleic acid, dispersive fine particles carrying previously bound receptors may be used instead. The present step may be carried out before or after the step of preparing the target nucleic acid.

Hereinafter, the step of binding the target nucleic acid to the fine particles will be described. The target nucleic acid having the bound ligand described above and first and the second dispersive fine particles carrying a receptor are allowed to react with each other, forming bonds. The reaction may be performed by mixing a solution containing the target nucleic acid with two solutions containing the respective dispersive fine particles, or alternatively, stepwise by mixing respective dispersive fine particles one by one.

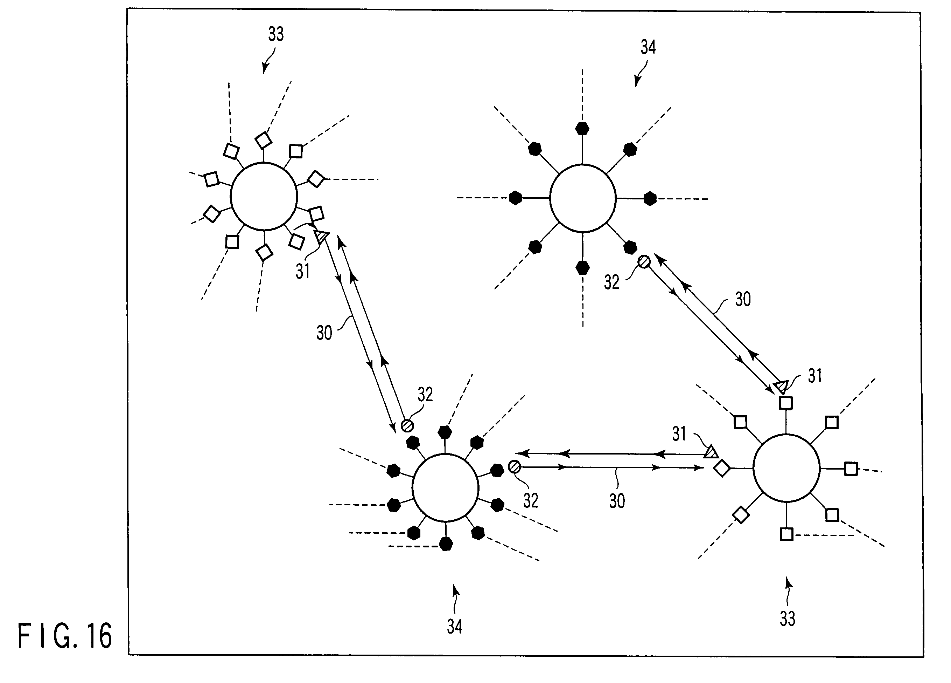

FIG. 16 is a schematic view showing the binding between the target nucleic acid and the fine particle. In FIG. 16, the first ligand 31 bound to the target nucleic acid 30 binds to the first fine particles 33. Also, the second ligand 32 bound to the target nucleic acid 30 binds to the second fine particle 34. Thus, one target nucleic acid binds to the first fine particle and also to the second fine particle, connecting the first and second fine particles to each other. In addition, binding of multiple target nucleic acids to one fine particle and of the respective target nucleic acids to other fine particles leads to binding of multiple fine particles to each other and hence, to aggregation of the fine particles. In FIG. 16, the dotted line extending from the receptor represents a nucleic acid schematically.

In this way, a target nucleic acid, if present in a sample, leads to aggregation of the fine particles, allowing detection of the target nucleic acid. The aggregation of fine particles may be detected by identifying dispersion of an optical signal from an optical marker additionally labeled as described above, but preferably by measurement of absorbance by a spectrophotometric method without use of any optical marker.

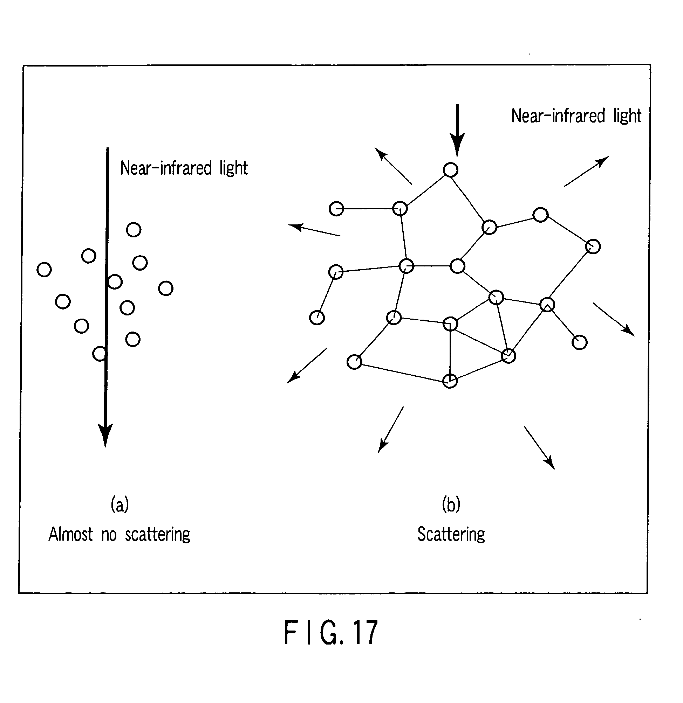

As shown in the schematic view of FIG. 17, a single fine particle hardly scatters near-infrared light (FIG. 17a). On the other hand, aggregated fine particles, which have a larger apparent diameter than that of single fine particle, cause scattering of near-infrared light (FIG. 17b). Accordingly, it is possible to determine the degree of aggregation of fine particles, by measuring the intensity of the transmission light from an incident beam in the near-infrared region by a photometer. Fine particles having a particle diameter of 300 nm or less causes scattering of near-infrared light, when aggregated, and thus use of fine particles having a particle diameter of 300 nm or less is preferable.

The solution containing the fine particles is preferably prepared in such a manner that it has a final concentration after mixing (before aggregation) in the range of 0.01 to 1 as OD and in the range of 3 or less as an OD after aggregation. In mixing the solution containing the target nucleic acid with the solution containing fine particles, the volume of one solution is preferably not less than 20% of the volume of the other solution, for prevention of localization of the ligand/receptor bonds, and the mixing is preferably performed rapidly, as the solution is stirred with a mixed latex mixer or by pipetting. The buffer for use in the reaction system is preferably a solution accelerating aggregation. When a different buffer is used, the fine particles are preferably washed twice or thrice with the different buffer before use.

When an antigen-antibody combination is used as the ligand-receptor combination, the reaction is preferably performed at a temperature of 0°C or higher and 37°C or lower for 5 to 30 minutes. The other ligand-receptor combination may be used as needed at a temperature for a period by using a buffer suitable for the reaction.

Unaggregated fine particles after mixing, which do not cause scattering of near-infrared light, do not require B/F separation before spectrophotometric measurement of the reaction solution, and the reaction system can be used directly for measurement. When the target nucleic acid is amplified by PCR, ligand-bound unreacted primers, unreacted nucleotides and others, if they may cause measurement error, may be separated. In separation, for example, biotin is bound to one ligand, and the resulting complex is B/F separated by using avidin- or streptoavidin-bound magnetic particles. The magnetic particles may be used then as the dispersive fine particles.

### [Method of determining the amount of nucleic acid quantitatively]

Hereinafter, the method of determining the amount of the nucleic acid quantitatively by using the method according to the present invention will be described. In quantitative determination, solutions containing the target nucleic acid at various concentrations are prepared by using the ligand-bound target nucleic acid described above. The nucleic acid solution at each concentration is allowed to react with the fine particles according to the method described above, and the absorbance of the solution is determined by a spectrophotometric method. A calibration curve for the relationship between the nucleic acid concentration and the absorbance is drawn, based on the measurement results. According to the calibration curve, the nucleic acid concentration is determined from the measurement results of the absorbance of the target nucleic acid to be detected. In this way, it is possible to determine the amount of the target nucleic acid quantitatively.

In a sample containing multiple kinds of target nucleic acids, it is possible to detect desirable target nucleic acids simultaneously in a sample containing multiple kinds of target nucleic acids, by binding two kinds of ligands different in kind to each of the target nucleic acids according to the kind thereof and supplying to the reaction only dispersive fine particles having the receptor specifically binding to the ligand bound to the target nucleic acid to be detected.

All ligands bound to the nucleic acid may be different from each other. Alternatively, one of the two kinds of ligands may be a ligand common to the other nucleic acid.

Two kinds of ligands were used to one target nucleic acid in the detection method described above, but three or more kinds of ligands may be used instead, and dispersive fine particles carrying an immobilized receptor specifically binding to each kind of ligand may be used.

### [Method of detecting mutation in the nucleotide sequence of nucleic acid]

Hereinafter, the method of detecting mutation in the nucleotide sequence of nucleic acid according to the present invention will be described. Nucleotide mutation includes single-nucleotide polymorphism (SNP), deletion, insertion and the like. The SNPs (Single Nucleotide Polymorphisms) is nucleotide diversity different individually that is often found on genome DNA.

In a first aspect of the present invention, provided is a method of detecting nucleic acid mutation by using a PCR-SSP method. The PCR-SSP method is a method of performing PCR by using a DNA polymerase, but the DNA polymerase shows almost no activity when the primer 3'-terminal is not hybridized completely with the template DNA.

Accordingly, when a primer having a sequence complementary to the standard target nucleotide sequence is used and there is an amplification product generated by PCR, there is no mutation, and, on the contrary when there is no amplification product, there is mutation.

One primer used in the method is a first ligand-bound detection primer having a sequence complementary to the target nucleotide sequence and having its 3'-terminal corresponding to the mutation site in the target nucleotide sequence. The other primer is a common primer having a bound second ligand, which is different from the first ligand, and has a sequence complementary to the 5'-terminal-sided sequence of the target nucleic acid.

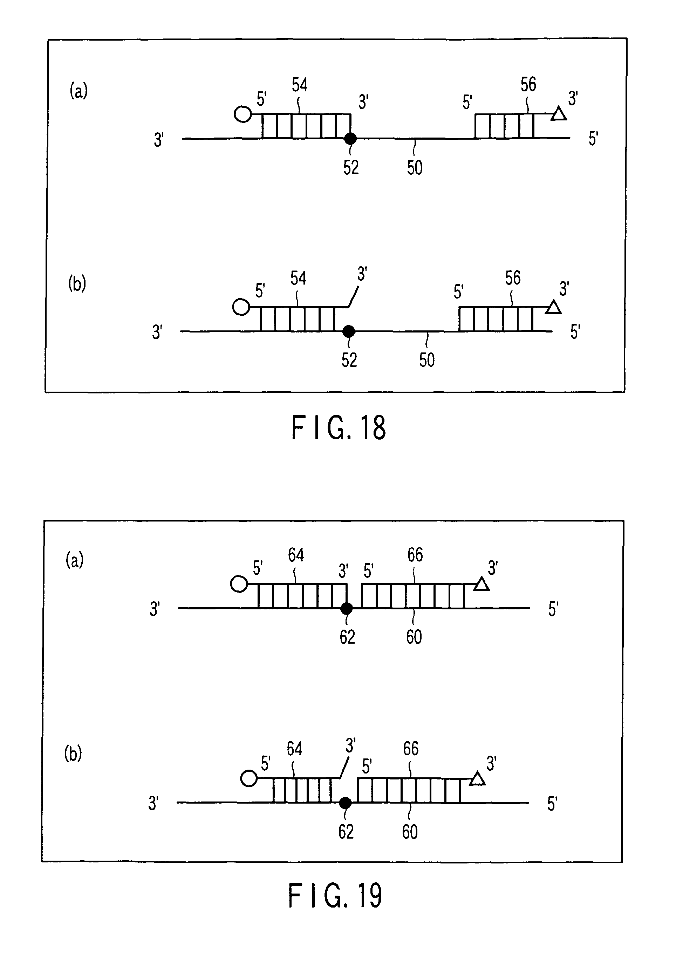

FIG. 18 is a schematic diagram showing the present aspect. FIG. 18a shows a target nucleic acid 50 without mutation, i.e., standard target nucleic acid 50, wherein the region possibly containing mutation is indicated by a black circle 52. In FIG. 18a, the detection primer 54 is hybridized completely, and gives an amplification product by PCR with the common primer 56. On the other hand, FIG. 18b shows a variant target nucleic acid 50. In this case, the detection primer 54 is not hybridized at the 3'-terminal, leading to no amplification by PCR.

Hereinafter, the steps in the present aspect will be described. First, in step (i), PCR is performed by using the target nucleic acid and the two kinds of primers 54 and 56. In the PCR, as described above, the amplification product is obtained only when the detection primer 54 is hybridized completely with the target nucleic acid. The common primer 56 is hybridized with the target nucleic acid, independently of the presence of mutation.

Then in step (ii), the PCR product obtained in the step above, the first dispersive fine particles having multiple receptors bound thereto, binding to the first ligand specifically, and the second dispersive fine particles having multiple receptors bound thereto, binding specifically to the second ligand, are allowed to react with each other.

Further, in step (iii), the aggregate in such a manner that the first and second dispersive fine particles are mutually linked when the PCR product is bound to the first and second dispersive fine particles, and a large number of the particles are mutually linked when two or more PCR products are bound to one of the particles, and simultaneously bound to the other particles respectively, is measured by a spectrophotometry.

These steps (ii) and (iii) are carried out according to the detection method by using dispersive fine particles described above in detail. Then, in step (iv), amplification of the nucleic acid by PCR is examined from the measurement results. Aggregation of fine particles indicates generation of an amplification product. Accordingly, aggregation indicates that there is no mutation in the target nucleic acid.

It is possible to detect mutation such as single-nucleotide polymorphism (SNP), deletion, or insertion in a nucleotide sequence easily and rapidly, according to the present aspect described above.

When the mutation in the target nucleic acid sequence is a single-nucleotide polymorphism, in another embodiment of the present aspect, a detection primer common for the standard and variant target nucleic acids may be used. Specifically, a first primer having a bound ligand for a standard target nucleic acid and having a sequence complementary to the standard target nucleic acid, and a second primer having a bound ligand for a variant target nucleic acid and having a sequence complementary to the variant target nucleic acid are used as the detection primers. These two kinds of detection primers may be added to the PCR system separately, or the PCR may be conducted simultaneously in a single reaction system.

In the present embodiment, fine particles corresponding to the ligands bound to the respective primers are used as the first dispersive fine particles. Specifically, dispersive fine particles for standard target nucleic acid carrying multiple receptors specifically binding to the ligand for standard target nucleic acid, and dispersive fine particles for variant target nucleic acid carrying multiple receptors specifically binding to the ligand for variant target nucleic acid are used.

Accordingly in the present embodiment, the coagulation reaction in the step (iii) in the first aspect is carried out separately with the fine particles for a standard target nucleic acid and with the fine particles for a variant target nucleic acid. There is one or both of the PCR products generated by the standard-target primer and by the variant-target primer contained in the PCR reaction solution.

Accordingly, the fine particles for a standard target nucleic acid and the second dispersive fine particles are added in combination to the reaction solution for a coagulation reaction. The aggregation, if it occurs in the reaction, indicates that the target nucleic acid is the standard type. Separately, the fine particles for the variant target nucleic acid and the second dispersive fine particles are used in combination for a coagulation reaction. The aggregation, if it occurs in the reaction, indicates that the target nucleic acid is a variant type. Further, aggregation in both coagulation reactions indicates that the target nucleic acid includes the standard and the variant type nucleic acids. Such a phenomenon may occur, for example, when a genome DNA of an individual is used as the sample and the genotype in the single-nucleotide polymorphism is hetero.

According to the present embodiment, it is possible to determine the nucleotide type in a single-nucleotide polymorphism merely by a single PCR reaction and also to determine the nucleotide type extremely easily and accurately because there is no need for a B/F separation operation.

In the present aspect, the ligand was bound to the primer, but may be bound to the nucleotide instead. PCR by using a ligand-bound nucleotide gives an amplification product carrying the ligand bound thereto.

It is also possible to use the method in the present aspect, for example, in detecting multiple mutation sites simultaneously by using for example a genome DNA as the sample.

In the second aspect of the present invention, provided is a method of detecting nucleic acid mutation by using a PCR-SSP method and a Tag sequence. The Tag sequence is an artificially designed nucleotide sequence that is preferably not present in biological samples such as chromosomes, is resistant to mishybridization, and has a dissociation temperature at a particular constant temperature. The Tag sequence is designed and prepared according to each target mutation site.

In the present aspect, a Tag sequence is bound, replacing the ligand above, to the detection primer in the first aspect. The Tag sequence is desirably bound to the 5' terminal of the detection primer. In addition, biotin is bound, replacing the ligand, to the common primer in the first aspect.

First, in the step (i) of the present aspect, PCR is carried out by using the target nucleic acid and the two kinds of primers. In the PCR, as described in the first aspect, an amplification product is generated only when the detection primer is completely hybridized with the target nucleic acid, .i.e., when there is no mutation in the target nucleic acid.

Subsequently, in step (ii), the biotin-bound PCR product is separated from the PCR reaction solution obtained in the step (i), by using avidin- or streptoavidin-bound magnetic particles. Because biotin binds to avidin or streptoavidin, the amplification product is bound to the magnetic particles via the bond, and thus it is possible to separate the magnetic particles from the PCR reaction solution by using magnetic force.

Then, in step (iii), the PCR product thus separated is added to PCR using a first ligand-bound first primer and a second ligand-bound second primer, both having a sequence complementary to the Tag sequence. In this way, the amplification product containing the Tag sequence is obtained. When there is no mutation in the target nucleic acid, the amplification product of the primer is generated, and the amplification product containing the Tag sequence is generated therefrom. Thus, the sequence of the mutation site is apparently converted to the Tag sequence. As described above, the Tag sequence is a sequence designed to be amplified easily and accurately by PCR, and thus the conversion proceeds smoothly.

The amplification product of the Tag sequence contains nucleotides having the first and second ligands bound thereto. Thus in step (iv), reaction with the first dispersive fine particles carrying multiple receptors binding to the first ligand specifically and the second dispersive fine particles carrying multiple receptors binding to the second ligand specifically results in aggregation, as described above. In step (v), the aggregation is analyzed by a spectrophotometric method. Finally, in step (vi), amplification of the Tag sequence by PCR is judged from the measurement results. Accordingly, amplification of the Tag sequence indicates that there is no mutation in the target nucleic acid.

The method in the present aspect described above can be used, for example, in detecting multiple mutation sites simultaneously by using, for example, a genome DNA as the sample. It is also possible to detect multiple mutation sites simultaneously, by using such a Tag sequence without restriction on the kind of the ligand.

The method of the present aspect has been described assuming that the target nucleic acid has no mutation, but it is also possible to use the method similarly, for example, in detecting a variant type of single-nucleotide polymorphism. In such a case, it is possible to detect similarly, by using a primer having a sequence complementary to the variant target nucleic acid as the detection primer.

In the third aspect of the present invention, provided is a method of detecting nucleotide mutation by ligation. The nucleic acid mutation detected in the present aspect is a single-nucleotide polymorphism (SNP). In the present aspect, a first ligand-bound detection primer having a sequence complementary to the target nucleic acid sequence and having its 3'-terminal corresponding to the single-nucleotide polymorphism site, and a second ligand-bound common primer having a sequence complementary to the 5'-terminal-sided sequence of the target nucleic acid polymorphism site and having its 5' terminal corresponding to the nucleotides close to the polymorphism site are used.

FIG. 19 is a schematic diagram showing the present aspect. FIG. 19a shows a target nucleic acid 60 without mutation, i.e., standard target nucleic acid, wherein the site possibly with mutation is indicated by a black circle 62. In FIG. 19a, the detection primer 64 is completely hybridized, and the 3'-terminal is close to the 5' terminal of the common primer 66. Accordingly, the two primers are connected to each other, when subjected to ligation reaction. On the other hand, FIG. 19b shows a variant target nucleic acid. In this case, the detection primer 64 is not hybridized at the 3'-terminal, prohibiting ligation reaction. The common primer 66 is hybridized with the target nucleic acid independently of the presence of mutation.

Hereinafter, the steps in the present aspect will be described step by step. First, in step (i), the two kinds of primers 64 and 66 are hybridized with the target nucleic acid 60. Subsequently, in step (ii), the two kinds of primers are connected to each other by ligation. In step (iii), the connected primer thus obtained is then allowed to react with first dispersive fine particles carrying multiple receptors binding to the first ligand specifically, and second dispersive fine particles carrying multiple receptors binding to the second ligand specifically.

In step (iv), the aggregate generated by the reaction is measured by a spectrophotometric method; and in step (v), amplification of the nucleic acid by the PCR is judged from the measurement results, and the single-nucleotide polymorphism in the target nucleic acid sequence is analyzed with the judgment results.

According to the present aspect, it is possible to analyze the presence of mutation by ligation reaction without PCR. Thus, the reaction step is more simplified, and the cost and the time can be reduced. However, only the sequences upstream and downstream of the mutation site can be used for the probe, and thus there is some restriction on the available sequence. For example when a DNA genome sequence is used as the sample, the region including the target mutation site may be amplified by PCR and then cleaved. In this way, it is also possible to improve the detection sensitivity and accuracy in the present aspect.

In the present aspect, the sites of the primer to which the ligand is bound and the number thereof are not particularly limited, but, for prevention of inhibition of ligase reaction, the ligand is preferably bound to a region not close to the mutation site, for example, to the terminal opposite to the mutation site.

In another aspect of the present invention, a detection primer common for the standard and variant target nucleic acids may be used. Specifically, a first primer having a bound ligand for a standard target nucleic acid and having a sequence complementary to the standard target nucleic acid and a second primer having a bound ligand for a variant target nucleic acid and having a sequence complementary to the variant target nucleic acid are used as the detection primers. These two kinds of detection primers may be subjected separately to ligation reaction, but the reaction may be carried out simultaneously in a single reaction system.

In such an embodiment, fine particles corresponding to the ligand bound to the respective primers are used as the first dispersive fine particles. Dispersive fine particles for standard target nucleic acid having multiple receptors binding to the ligand for standard target nucleic acid specifically, and dispersive fine particles for variant target nucleic acid having multiple receptors binding to the ligand for variant target nucleic acid specifically are used.

Accordingly in the embodiment, the coagulation reaction in the step (iii) is carried out separately with the fine particles for a standard target nucleic acid and with the fine particles for a variant target nucleic acid. There is one or both of the products due to the standard-target primer and due to the variant-target primer contained in the reaction solution after ligation. The coagulation reaction is carried out by adding the fine particles for a standard target nucleic acid and the second dispersive fine particles in combination in the reaction solution. Aggregation in the reaction, if it occurs, indicates that the target nucleic acid is the standard nucleic acid. Separately, the coagulation reaction is carried out by using the fine particles for a variant target nucleic acid and the second dispersive fine particles in combination. Aggregation in the reaction indicates that the target nucleic acid is a variant-type nucleic acid. Further, aggregation in both coagulation reactions, if it occurs, indicates that the target nucleic acid includes both the standard- and variant-type nucleic acids. Such a phenomenon may occur, for example, when a genome DNA of an individual is used as the sample and the genotype in the single-nucleotide polymorphism is hetero.

According to the present embodiment, it is possible to determine the nucleotide type in a single-nucleotide polymorphism merely by a single ligation reaction and to determine the nucleotide type extremely easily and accurately because there is no need for a B/F separation operation.

In the fourth aspect of the present invention, provided is a method of detecting nucleic acid mutation by ligation and by using a Tag sequence. The nucleotide mutation detected in the present aspect is a single-nucleotide polymorphism.

In the present aspect, a Tag sequence is used similarly to the second aspect above. The mutation is detected by using a ligation reaction, similarly to the third aspect.

Specifically, a detection primer having a sequence complementary to the target nucleic acid sequence and having its 3'-terminal corresponding to the mutation site of the target nucleic acid sequence and its 5' terminal having a Tag sequence bound thereto, and a common primer having a sequence complementary to the 5'-terminal-sided sequence of the target nucleic acid mutation site and having biotin bound thereto are used. The detection method comprises:
a step (i) of hybridizing the two kinds of primers with the target nucleic acid;
a step (ii) of ligating the hybrid and connecting the two kinds of primers to each other after the step (i);
a step (iii) of separating the biotin-bound primer from the reaction system in the step (ii) by using avidin- or streptoavidin-bound magnetic particles;
a step (iv) of obtaining the PCR product of the Tag sequence by performing PCR by using the connected primer obtained in the step (iii) and the first ligand-bound first primer having a sequence complementary to the Tag sequence and the second ligand-bound second primer;
a step (v) of allowing the PCR product obtained in the step (iv), first dispersive fine particles carrying multiple receptors binding to the first ligand specifically, and second dispersive fine particles carrying multiple receptors binding to the second ligand specifically to react with each other;
a step (vi) of measuring the aggregate in such a manner that the first and second dispersive fine particles are mutually linked when the PCR product is bound to the first and second dispersive fine particles, and a large number of the particles are mutually linked when two or more PCR products are bound to one of the particles, and simultaneously bound to the other particles respectively, by a spectrophotometry; and
a step (vii) of judging amplification of the nucleic acid by the PCR from the measurement results and detecting the mutation in the target nucleotide sequence with the judgment result.

The method in the present aspect can be used in detecting multiple mutation sites simultaneously, for example, by using a genome DNA as the sample. It is also possible to detect multiple mutation sites simultaneously, by using such a Tag sequence without restriction on the kind of the ligand. It is also possible to analyze the presence of mutation by ligation reaction without PCR. Thus, the reaction step is more simplified, and the cost and the time can be reduced.

The various aspects according to the present invention described above can be used in quantitative determination of the target nucleic acid. In quantitative determination, a solution containing the target nucleic acid to be detected at a known concentration is prepared and analyzed similarly to the methods above. The analysis is repeated with nucleic acid solutions different in concentration, and a calibration curve showing the relationship between the nucleic acid concentration and the absorbance is drawn, based on the measurement results. According to the calibration curve, the nucleic acid concentration is determined from the measurement results of the absorbance of the target nucleic acid to be detected. In this way, it is possible to determine the amount of the target nucleic acid quantitatively.

In the various aspects according to the present invention described above, it is possible to detect many mutation sites contained in a sample containing multiple kinds of target nucleic acids and also in a desirable target nucleic acid simultaneously. It is possible to detect mutation at multiple sites easily and simultaneously, by performing PCR and ligation reaction simultaneously with a sample having mutations at multiple sites, dividing the reaction solution into portions, and performing a coagulation reaction with respective portions by using dispersive fine particles for detection of respective mutation sites.

Two kinds of ligands were used for one target nucleic acid in the detection methods described above, but three or more kinds of ligands may be used instead, and dispersive fine particles carrying an immobilized receptor specifically binding to each kind of ligand may also be used.

As described above in detail, according to the method of the present invention, it is possible to detect a desirable nucleic acid easily, rapidly, and accurately. Therefore, the method is effective, for example, for simplification and acceleration of clinical tests, and applicable to a wide range of applications, including simple tests to full automatic assays.

Examples of the materials used in performing the method according to the present invention include 300-mm carboxy-type latex particles (CMP) G0201 (0.104 meq COO/g) (manufactured by JSR), EDAC (manufactured by Sigma), rabbit anti-biotin antibody (BETHYL Laboratory Inc.), rabbit anti-digoxigenin (Dig.) antibody (Roche diagnostic corp.), template DNA, 5' biotinated SD primer, 3' Diog. ED primer, Titanium taq DNA polymerase (manufactured by BD), and MES (manufactured by WAKO). Examples of the devices for use include a rotator, Voltex mixer, tangential flow system, dialysis membrane, magnetic stirrer, spectrophotometer, cell, and the like.

The methods I and II described above are both higher in reactivity and also in specificity. In addition, because multiplex reactions proceed smoothly, the method is favorably used in solution-based reactions by using cubets or microwells. Accordingly, the present invention provides a detection method most suitable for high-throughput automatic analyzers involving liquid handling with an aliquotting nozzle.

### Industrial Applicability

The method for detecting a nucleic acid according to the present invention is used in tests for determination of polymorphism, mutation, and the expression amount of a nucleic acid. In particular, the method for detecting a nucleic acid according to the present invention is used for detection of SNPs. The method for detecting a nucleic acid according to the present invention will be useful in showing the SNPs and the pathologic genetic background of an individual, which, in turn, enables development of a medicine personalized to the individual.

## Claims

1. A method for detecting a nucleic acid, the method comprising:
(1) binding first and second ligands to a nucleic acid to be detected;
(2) in the nucleic acid to which the ligands have been bound, binding the first ligand to a solid phase carrier supporting receptors specifically binding to the first ligand, and obtaining a complex of the nucleic acid - solid phase carrier;
(3) in the complex, binding the second ligand to a receptor modified with a labeling substance specifically binding to the second ligand; and
(4) detecting the nucleic acid by detecting the labeling substance.

2. The method for detecting a nucleic acid according to claim 1, wherein the first ligand is the same as or different from the second ligand.

3. A method for detecting two or more kinds of nucleic acids to be detected, the method comprising:
(1) binding first and second ligands to the two or more kinds of nucleic acids so that the kinds of the first ligand are different for each kind of the nucleic acids;
(2) preparing solid phase carriers supporting receptors specifically binding to the first ligand for each kind of the first ligand, binding the first ligands to the solid phase carrier corresponding to each first ligand, and separating the nucleic acid for each kind of the first ligand;
(3) binding a receptor modified with a labeling substance specifically binding to the second ligand; and
(4) detecting the two or more kinds of nucleic acids by detecting the labeling substance.

4. A method for simultaneously detecting two or more kinds of nucleic acids to be detected, the method comprising:
(1) binding first and second ligands to the two or more kinds of nucleic acids so that the kinds of the second ligand are different for each kind of the nucleic acids;
(2) binding the first ligand to a solid phase carrier supporting receptors specifically binding to the first ligand, and obtaining a complex of the nucleic acid - solid phase carrier;
(3) preparing two or more kinds of receptors modified with a different labeling substance for each kind of ligand, and binding the receptors to each second ligand corresponding to each kind of receptor respectively;
(4) simultaneously detecting two or more kinds of nucleic acids by detecting the different labeling substance, respectively.

5. The method for detecting a nucleic acid according to claim 1-4, in binding the ligands to the nucleic acid to be detected, the method comprising:
adding to the sample containing the nucleic acid, the test reagent containing the first nucleic acid primer to which the first ligand is attached and having the base corresponding to mutation site of the nucleic acid at the end of elongating site and the second nucleic acid primer to which the second ligand is attached, the nucleic acid being amplified by polymerase chain reaction of the first and second nucleic acid primer; and
obtaining the amplified nucleic acid to which the first and second ligands are attached by polymerase chain reaction.

6. The method for detecting a nucleic acid according to claim 1-4, in binding the ligands to the nucleic acid to be detected, the method comprising:
adding to the sample containing the nucleic acid, the test reagent containing the first nucleic acid probe to which the first ligand is attached and having the base corresponding to mutation site of the nucleic acid at the end of binding site and the second nucleic acid probe to which the second ligand is attached, a ligating reaction with the first nucleic acid probe being possible on the nucleic acid;
hybridizing the first and second nucleic acid probes to the nucleic acid; and
binding between the first and second nucleic acid probes by ligating reaction, and obtaining the nucleic acid to which the first and second ligands are attached.

7. The method for detecting a nucleic acid according to claim 6, before the ligating reaction is performed, amplifying in advance the region containing the site of the nucleic acid in which the ligating reaction is performed, by polymerase chain reaction.

8. A method for detecting a nucleic acid containing the mutation site and/or the nucleic acid containing the standard site for detection in the sample of nucleic acid, the method comprising:
(1) adding to the nucleic acid sample, the test reagent containing (i) the first primer to which the first ligand is attached, the first primer having the base corresponding to mutation site of the nucleic acid at the end of the elongating site, and (ii) the second primer to which the second ligand is attached, the second primer having the base corresponding to standard site of the nucleic acid at the end of the elongating site, and not having the base corresponding to mutation site of the nucleic acid at the end of the elongating site, and (iii) the third primer to which the third ligand is attached, the nucleic acid being amplified by polymerase chain reaction of the first to the third primer;
(2) amplifying the nucleic acid containing the mutation site between the first primer and the third primer, and/or amplifying the nucleic acid containing the standard site between the second primer and the third primer by polymerase chain reaction, then obtaining the nucleic acid;
(3) preparing solid phase carriers supporting receptors specifically binding to the first or the second ligand respectively, binding the first or the second ligand to the solid phase carrier corresponding to first or second ligand respectively, and separating the nucleic acid containing the mutation site modified with the first ligand and the nucleic acid containing the standard site modified with the second ligand;
(4) in nucleic acid to which the solid phase carrier is bound, binding the receptor modified with the labeling substance specifically binding to the third ligand to the third ligand; and
(5) detecting the nucleic acid containing the mutation site and/or the nucleic acid containing the standard site by detecting the labeling substance.

9. A method for detecting a nucleic acid containing the mutation site and/or the nucleic acid containing the standard site for detection in the sample of nucleic acid, the method comprising:
(1) adding to the nucleic acid sample, the test reagent containing (i) the first nucleic acid probe to which the first ligand is attached, the first nucleic acid probe having the base corresponding to mutation site of the nucleic acid at the end of the binding site, and (ii) the second nucleic acid probe to which the second ligand is attached, the second nucleic acid probe having the base corresponding to the standard site of the nucleic acid at the end of the binding site, and not having the base corresponding to the mutation site of the nucleic acid at the end of the binding site, and (iii) the third nucleic acid probe to which the third ligand is attached, a ligating reaction of the first to the third nucleic acid probe is possible on the nucleic acid;
(2) hybridizing the first and the third nucleic acid probe to the nucleic acid containing the mutation site, and/or hybridizing the second and the third nucleic acid probe to the nucleic acid containing the standard site;
(3) binding between the first and the third nucleic acid probe by ligating reaction, and/or binding between the second and the third nucleic acid probe by ligating reaction, then obtaining the nucleic acid containing the mutation site modified with the first and the third ligand and/or obtaining the nucleic acid containing the standard site modified with the second and the third ligand;
(4) preparing solid phase carriers supporting receptors specifically binding to the first or the second ligand respectively, binding the first or the second ligand to the solid phase carrier corresponding to first or second ligand respectively, and separating the nucleic acid containing the mutation site modified with the first ligand and the nucleic acid containing the standard site modified with the second ligand;
(5) in nucleic acid to which the solid phase carrier is bound, binding the receptor modified with the labeling substance specifically binding to the third ligand to the third ligand; and
(6) detecting the nucleic acid containing the mutation site and/or the nucleic acid containing the standard site by detecting the labeling substance.

10. The method for detecting a nucleic acid according to claim 9, before the ligating reaction is performed, amplifying in advance the region containing the site of the nucleic acid in which the ligating reaction is performed.

11. A method for detecting a nucleic acid containing the mutation site and/or the nucleic acid containing the standard site for detection in the sample of nucleic acid, the method comprising:
(1) adding to the nucleic acid sample, the test reagent containing (i) the first primer to which the first ligand is attached, the first primer having the base corresponding to mutation site of the nucleic acid at the end of the elongating site, and (ii) the second primer to which the second ligand is attached, the second primer having the base corresponding to standard site of the nucleic acid at the end of the elongating site, and not having the base corresponding to mutation site of the nucleic acid at the end of the elongating site, and (iii) the third primer to which the third ligand is attached, the nucleic acid being amplified by polymerase chain reaction of the first to the third primer;
(2) amplifying the nucleic acid containing the mutation site between the first primer and the third primer by polymerase chain reaction, and/or amplifying the nucleic acid containing the standard site between the second primer and the third primer by polymerase chain reaction, then obtaining the amplified nucleic acid containing the mutation site modified with the first and the third ligand and/or obtaining the amplified nucleic acid containing the standard site modified with the second and the third ligand;
(3) binding the third ligand to the solid phase carrier supporting receptors specifically binding to the third ligand, then obtaining a complex of the nucleic acid - solid phase carrier;
(4) preparing two kinds of the receptors modified with the different labeling substances specifically binding to the first or the second ligand respectively, and binding the receptors to each ligand respectively; and
(5) simultaneously detecting the nucleic acid containing the mutation site and/or the nucleic acid containing the standard site for detection by detecting the different labeling substance respectively.

12. A method for detecting a nucleic acid containing the mutation site and/or a nucleic acid containing the standard site for detection in the sample of nucleic acid, the method comprising:
(1) adding to the nucleic acid sample, the test reagent containing (i) the first nucleic acid probe to which the first ligand is attached, the first nucleic acid probe having the base corresponding to mutation site of the nucleic acid at the end of the binding site, and (ii) the second nucleic acid probe to which the second ligand is attached, the second nucleic acid probe having the base corresponding to the standard site of the nucleic acid at the end of the binding site, and not having the base corresponding to the mutation site of the nucleic acid at the end of the binding site, and (iii) the third nucleic acid probe to which the third ligand is attached, a ligating reaction of the first to the third nucleic acid probe is possible on the nucleic acid;
(2) hybridizing the first and the third nucleic acid probe to the nucleic acid containing the mutation site, and/or hybridizing the second and the third nucleic acid probe to the nucleic acid containing the standard site;
(3) binding between the first and the third nucleic acid probe by ligating reaction, and/or binding between the second and the third nucleic acid probe by ligating reaction, then obtaining the nucleic acid containing the mutation site modified with the first and the third ligand and/or obtaining the nucleic acid containing the standard site modified with the second and the third ligand;
(4) binding the third ligand to the solid phase carrier supporting receptors specifically binding to the third ligand, and obtaining a complex of the nucleic acid - solid phase carrier;
(5) preparing two kinds of the receptors modified with the different labeling substances specifically binding to the first or the second ligand respectively, and binding the receptors to each ligand respectively; and
(6) simultaneously detecting the nucleic acid containing the mutation site and/or the nucleic acid containing the standard site for detection by detecting the different labeling substance respectively.

13. The method for detecting a nucleic acid according to claim 12, before the ligating reaction is performed, amplifying in advance the region containing the site of the nucleic acid in which the ligating reaction is performed, by polymerase chain reaction.

14. The method for detecting a nucleic acid according to claim 1-4, 8-10, **characterized in that** the solid phase carrier is magnetic particles, the magnetic particles being aggregated in one place by applying magnetism.

15. The method for detecting a nucleic acid according to claim 1-4, 8-10, wherein the ligand and the receptor is an antigens or an antibody, the specific bond between the ligand and receptor is performed by antigen-antibody reaction.

16. A method for detecting a target nucleic acid based on an aggregate generated by crosslinking reaction between the target nucleic acid to be detected and dispersive fine particles, the method comprising:
(1) reacting the target nucleic acid to which different kinds of first and second ligands have been attached, with first particles to which two or more receptors specifically binding to the first ligand have been attached and second particles to which two or more receptors specifically binding to the second ligand have been attached; and
(2) obtaining the aggregate in such a manner that the first and second particles are mutually linked when the target nucleic acid is bound to the first and second particles, and a large number of the particles are mutually linked when two or more target nucleic acids are bound to one of the particles, and simultaneously bound to the other particles respectively, and measuring the aggregate by spectrophotometry.

17. The method for detecting a target nucleic acid according to claim 16, further comprising:
(1) reacting the target nucleic acid whose concentration is known, to which two or more the first and second ligands are bound respectively, to the first and second particles, and measuring the aggregate obtained by the reaction by spectrophotometry;
(2) performing similarly the step of (1) about the target nucleic acid of concentration which is different from the step of (1), and creating a calibration curve from the result of the measurements thereof;
(3) reacting the target nucleic acid for detection to the first and second particles, and measuring the aggregate obtained by the reaction by spectrophotometry; and
(4) determining the concentration of the target nucleic acid for detection on the basis of the calibration curve.

18. The method for detecting a target nucleic acid according to claim 16, wherein the target nucleic acid is the target nucleic acid to which three or more kinds of ligands are bound, and the dispersive fine particle is the dispersive fine particle to which receptors specifically binding to the three or more kinds of ligands respectively are bound.

19. The method for detecting a target nucleic acid according to claim 18, wherein the different one or two kinds of ligands are bound for each kind of the nucleic acid, and the dispersive fine particle is the dispersive fine particle to which the receptor is bound, the receptor specifically binding to ligand to which the target nucleic acid for detection is bound.

20. The method for detecting a target nucleic acid according to claim 19, wherein the dispersive fine particle is the latex particle whose diameter is 300 nm or less.

21. The method for detecting a target nucleic acid according to claim 16, wherein the ligand is selected from the group consisting of hydrophilic organic compound, dioxygen, fluorescein, alexa, polypeptide which the number of residues are 6 or more, carbohydrate chain which the number of carbohydrates are two or more, biotin, protein, polyhistidine, HA, GST, and Flag.

22. The method for detecting a target nucleic acid according to claim 16, wherein the target nucleic acid to which the ligands are attached, is prepared by polymerase chain reaction of the first primer to which the first ligand is attached and the second primer to which the second ligand is attached, the second ligand being different kind from the first ligand.

23. The method for detecting a target nucleic acid according to claim 16, wherein the target nucleic acid to which the ligands are attached, is prepared by polymerase chain reaction of a nucleotide to which the desired ligand is attached.
